Europäisches Patentamt
European Patent Office
Office européen des brevets

(19)

(11) Publication number: 0 252 682 A2

(12) EUROPEAN PATENT APPLICATION

(21) Application number: 87305846.5

(22) Date of filing: 01.07.87

(51) Int. Cl.4: C07D 487/04 , A61K 31/41 , //(C07D487/04,257:00,231:00),(-C07D487/04,257:00,235:00)

(30) Priority: 02.07.86 GB 8616125

(43) Date of publication of application: 13.01.88 Bulletin 88/02

(84) Designated Contracting States: AT BE CH DE ES FR GB GR IT LI LU NL SE

(71) Applicant: MAY & BAKER LIMITED

Dagenham Essex RM10 7XS(GB)

(72) Inventor: Stevens, Malcolm Fracis Graham
University of Aston
Birmingham(GB)
Inventor: Horspool, Keith Ronald
University of Aston
Birmingham(GB)
Inventor: Baig, Ghouse Unissa
University of Aston
Birmingham(GB)
Inventor: Lunt, Edward
May & Baker Limited
Dagenham Essex, RM10 7XS(GB)
Inventor: Newton, Christopher Gregory
May & Baker Limited
Dagenham Essex, RM10 7XS(GB)
Inventor: Walsh, Roger John Aitchison
May & Baker Limited
Dagenham Essex, RM10 7XS(GB)

(74) Representative: Bentham, Stephen et al
J.A. Kemp & Co. 14 South Square Gray's Inn
London WC1R 5EU(GB)

(54) **Tetrazines.**

(57) Tetrazines of the formula:

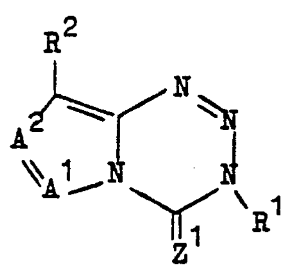

I

EP 0 252 682 A2

wherein $R^1$ represents cycloalkyl, optionally substituted alkyl, alkenyl or alkynyl, $A^1$ represents nitrogen or $-CR^3=$ wherein $R^3$ represents hydrogen or a substituent, $A^2$ represents nitrogen or, when $A^1$ represents nitrogen, $A^2$ represents nitrogen or a group $-CR^3=$ as hereinbefore defined, $Z^1$ represents oxygen or sulphur, and $R^2$ represents a cyano or azidocarbonyl group or a group of the formula $-COZ^2R^9$, $-CONHOR^9$, $-CONHNR^9R^{10}$, $-CONHNHCOOR^9$, $-CONHNHCONHR^9$ $-COCR^{11}=N_2$, $-CONHR^{12}$ or $-CON=S(O)R^{13}R^{14}$ wherein $Z^2$ is oxygen or sulphur and $R^9$ and $R^{10}$ each represents hydrogen, alkyl which may carry an optionally substituted phenyl substituent, or represents optionally substituted phenyl, $R^{11}$ represents hydrogen or alkyl, and $R^{12}$ represents optionally substituted alkyl and $R^{13}$ and $R^{14}$ each represent alkyl, and when $R^1$, $R^2$, or $R^3$ represents or contains an acidic group, salts thereof are useful as pharmaceuticals; processes for their preparation and new intermediates are described.

**"TETRAZINES"**

This invention relates to tetrazine derivatives, to processes for their preparation and to pharmaceutical compositions containing them.

The present invention provides the use of tetrazine derivatives of the general formula shown in Figure I of the drawings assembled at the end of the present specification [wherein $R^1$ represents a cycloalkyl group, or a straight-or branched-chain alkyl, alkenyl or alkynyl group containing up to 6 carbon atoms, each such alkyl, alkenyl or alkynyl group being unsubstituted or substituted by from one to three substituents selected from halogen (i.e. bromine, iodine or, preferably, chlorine or fluorine) atoms, straight-or branched-chain alkoxy, alkylthio, alkylsulphinyl and alkylsulphonyl groups each containing up to 4 carbon atoms, and optionally substituted phenyl groups, $A^1$ represents a nitrogen atom or a group $-CR^3=$ wherein $R^3$ represents a hydrogen atom or a substituent $R^4$ wherein $R^4$ represents a halogen atom, or a straight-or branched-chain alkyl or alkenyl group, containing up to 6 carbon atoms, which may carry up to 3 substituents selected from halogen atoms, optionally substituted phenyl groups, and straight-or branched-chain alkoxy, alkylthio and alkylsulphonyl groups containing up to 3 carbon atoms, or $R^4$ represents a cycloalkyl, cyano, hydroxy, nitro or optionally substituted phenoxy group of a group of the formula $-COR^5$ - (wherein $R^5$ represents an alkyl or alkoxy group of up to 4 carbon atoms, or a hydroxy group, or an optionally substituted phenyl group) or an alkanoylamino group containing up to 6 carbon atoms, or $R^4$ represents a group of the formula

$-S(O)_{\underline{n}}R^6$, $-SO_2NR^7R^8$ or $-CZ^2NR^7R^8$

(wherein $\underline{n}$ represents 0, 1 or 2, $R^6$ represents a straight-or branched-chain alkyl or alkenyl group, containing up to 4 carbon atoms, which may carry an optionally substituted phenyl substituent, a cycloalkyl group or an optionally substituted phenyl group, $R^7$ and $R^8$, which may be the same or different, each represents a hydrogen atom or a straight-or branched-chain alkyl or alkenyl group, containing up to 4 carbon atoms, which may carry an optionally substituted phenyl substituent, or a cycloalkyl group or an optionally substituted phenyl group or the group $-NR^7R^8$ represents a heterocyclic group, and $Z^2$ represents an oxygen or sulphur atom), $A^2$ represents a nitrogen atom or, when $A^1$ represents a nitrogen atom, $A^2$ represents a nitrogen atom or a group $-CR^3=$ wherein $R^3$ is as hereinbefore defined, $Z^1$ represents an oxygen or sulphur atom, and $R^2$ represents a cyano or azidocarbonyl group or a group of the formulae II to IX:-

$-COZ^2R^9$ II

$-CONHOR^9$ III

$-CONHNR^9R^{10}$ IV

$-CONHNHCOOR^9$ V

$-CONHNHCONHR^9$ VI

$-COCR^{11}=N_2$ VII

$-CONHR^{12}$ VIII or

$-CON=S(O)R^{13}R^{14}$ IX,

wherein $Z^2$ is as hereinbefore defined and $R^9$ and $R^{10}$ each represents a hydrogen atom or a straight-or branched-chain alkyl group containing from 1 to 4 carbon atoms, which may carry an optionally substituted phenyl substituent, or represents an optionally substituted phenyl group, $R^{11}$ represents a hydrogen atom or a straight-or branched-chain alkyl group containing from 1 to 4 carbon atoms, and $R^{12}$ represents a straight-or branched-chain alkyl group, containing from 1 to 4 carbon atoms, substituted by one or more halogen, e.g. chlorine, atoms or by a straight-or branched-chain alkoxycarbonyl group containing from 1 to 4 carbon atoms in the alkoxy moiety, or represents a straight-or branched-chain alkyl group containing 5 to 8 carbon atoms, optionally substituted by one or more halogen, e.g. chlorine, atoms and $R^{13}$ and $R^{14}$ each represent a straight-or branched-chain alkyl group containing from 1 to 4 carbon atoms, preferably methyl], and when $R^1$, $R^2$, or $R^3$ represents or contains an acidic group, salts thereof, more especially alkali metal, e.g. sodium, salts, for the preparation of a pharmaceutical product for the treatment of the human or animal body against carcinomas, melanomas, sarcomas, lymphomas and leukaemias, and for the treatment of organ grafts and skin grafts and for the treatment of immunological diseases.

Whenever the context so permits reference in this specification to the compounds of the general formula shown in Figure I is meant to include reference to the said salts. The salts are particularly useful as intermediates.

Within the definition of the formula shown in Figure I the optional substituents on the phenyl and phenoxy moieties may be selected from, for example, halogen atoms and alkyl and alkoxy groups containing up to 4 carbon atoms, and nitro groups. Cycloalkyl groups within the definition of the formula shown in Figure I contain 3 to 8, preferably 6, carbon atoms. A heterocyclic group within the definition of

the formula shown in Figure I is a 5-, 6-or 7-membered heterocyclic ring which may optionally contain a further heteroatom, i.e. nitrogen, oxygen or sulphur, and which may carry one of two straight-or branched-chain alkyl substituents each containing up to 4 carbon atoms, e.g. a piperidino group or a 2-methyl-, 3-methyl-, 4-methyl-, 2,4-dimethyl-, or 3,5-dimethylpiperidino group, or a morpholino, pyrrolidin-1-yl, perhydroazepin-1-yl, piperazin-1-yl, 4-methylpiperazin-1-yl or 1,4-thiazin-4-yl group.

The specification of West German Patent Application No. 2932305 describes compounds of the formula shown in Figure I wherein $R^1$ represents, inter alia, a cycloalkyl group, or a straight-or branched-chain alkyl group containing up to 6 carbon atoms, optionally substituted by halogen or alkoxy containing up to 3 carbon atoms, or represents a methyl or ethyl group substituted by a phenyl group, $R^2$ represents, inter alia, a cyano group or an alkoxycarbonyl group containing up to 5 carbon atoms, $A^1$ represents, inter alia, a nitrogen atom or a group $-CR^3=$ wherein $R^3$ represents a hydrogen atom or a substituent $R^4$ wherein $R^4$ represents an alkyl, cycloalkyl or benzyl group, $A^2$ represents, inter alia, a nitrogen atom or a group $-CR^3=$ wherein $R^3$ represents a hydrogen atom or a substituent $R^4$ wherein $R^4$ represents an alkyl, cycloalkyl or benzyl group; and $Z^1$ represents an oxygen atom: the compounds are described as being useful in the protection of plants, as herbicides or fungicides or as plant growth regulators; they are also stated to possess properties indicative of utility as diuretics, sedatives, tranquillisers and antifebrile agents; some of the compounds are said to be useful as dyestuffs or as intermediates for dyestuffs; nowhere in this document is suggested their valuable antineoplastic and immunomodulatory activities.

Thus, as a further feature of the invention, there are provided new tetrazine derivatives of the general formula shown in Figure I of the drawings assembled at the end of the present specification [wherein $R^1$ represents a cycloalkyl group, or a straight-or branched-chain alkyl, alkenyl or alkynyl group containing up to 6 carbon atoms, each such alkyl, alkenyl or alkynyl group being unsubstituted or substituted by from one to three substituents selected from halogen (i.e. bromine, iodine or, preferably, chlorine or fluorine) atoms, straight-or branched-chain alkoxy, alkylthio, alkylsulphinyl and alkylsulphonyl groups each containing up to 4 carbon atoms, and optionally substituted phenyl groups, $A^1$ represents a nitrogen atom or a group $-CR^3=$ wherein $R^3$ represents a hydrogen atom or a substituent $R^4$ wherein $R^4$ represents a halogen atom, or a straight-or branched-chain alkyl or alkenyl group, containing up to 6 carbon atoms, which may carry up to 3 substituents selected from halogen atoms, optionally substituted phenyl groups, and straight-10 branched-chain alkoxy, alkylthio and alkylsulphonyl groups containing up to 3 carbon atoms, or $R^4$ represents a cycloalkyl, cyano, hydroxy, nitro or optionally substituted phenoxy group or a group of the formula $-COR^5$ - (wherein $R^5$ represents an alkyl or alkoxy group of up to 4 carbon atoms, or a hydroxy group, or an optionally substituted phenyl group) or an alkanoylamino group containing up to 6 carbon atoms, or $R^4$ represents a group of the formula

$-S(O)_{\underline{n}}R^6$, $-SO_2NR^7R^8$ or $-CZ^2NR^7R^8$

(wherein n represents 0, 1 or 2, $R^6$ represents a straight-or branched-chain alkyl or alkenyl group, containing up to 4 carbon atoms, which may carry an optionally substituted phenyl substituent, a cycloalkyl group or an optionally substituted phenyl group, $R^7$ and $R^8$, which may be the same or different, each represents a hydrogen atom or a straight-or branched-chain alkyl or alkenyl group, containing up to 4 carbon atoms, which may carry an optionally substituted phenyl substituent, or a cycloalkyl group or an optionally substituted phenyl group or the group $-NR^7R^8$ represents a heterocyclic group, and $Z^2$ represents an oxygen or sulphur atom), $A^2$ represents a nitrogen atom or, when $A^1$ represents a nitrogen atom, $A^2$ represents a nitrogen atom or a group $-CR^3=$ wherein $R^3$ is as hereinbefore defined, $Z^1$ represents an oxygen or sulphur atom, and $R^2$ represents a cyano or azidocarbonyl group or a group of the formulae II to IX:-

$-COZ^2R^9$ II
$-CONHOR^9$ III
$-CONHNR^9R^{10}$ IV
$-CONHNHCOOR^9$ V
$-CONHNHCONHR^9$ VI
$-COCR^{11}=N_2$ VII
$-CONHR^{12}$ VIII or
$-CON=S(O)R^{13}R^{14}$ IX,

wherein $Z^2$ is as hereinbefore defined and $R^9$ and $R^{10}$ each represents a hydrogen atom or a straight-or branched-chain alkyl group containing from 1 to 4 carbon atoms, which may carry an optionally substituted phenyl substituent, or represents an optionally substituted phenyl group, $R^{11}$ represents a hydrogen atom or a straight-or branched-chain alkyl group containing from 1 to 4 carbon atoms, and $R^{12}$ represents a straight-or branched-chain alkyl group, containing from 1 to 4 carbon atoms, substituted by one or more halogen, e.g. chlorine, atoms or by a straight-or branched-chain alkoxycarbonyl group containing from 1 to 4 carbon atoms in the alkoxy moiety, or represents a straight-or branched-chain alkyl group containing 5 or more

carbon atoms, optionally substituted by one or more halogen, e.g. chlorine, atoms and $R^{13}$ and $R^{14}$ each represent a straight-or branched-chain alkyl group containing from 1 to 4 carbon atoms, preferably methyl], and when $R^1$, $R^2$, or $R^3$ represents or contains an acidic group, salts thereof, more especially alkali metal, e.g. sodium, salts, with the proviso that compounds wherein $R^1$ represents a cycloalkyl group, or a straight-or branched-chain alkyl group containing up to 6 carbon atoms, optionally substituted by halogen or alkoxy containing up to 3 carbon atoms, or represents a methyl or ethyl group substituted by a phenyl group, $R^2$ represents a cyano group of an alkoxycarbonyl group containing up to 5 carbon atoms, $A^1$ represents a nitrogen atom or a group $-CR^3=$ wherein $R^3$ represents a hydrogen atom or a substituent $R^4$ wherein $R^4$ represents an alkyl, cycloalkyl or benzyl group, $A^2$ represents a nitrogen atom or a group $-CR^3=$ wherein $R^3$ represents a hydrogen atom or a substituent $R^4$ wherein $R^4$ represents an alkyl, cycloalkyl or benzyl group, and $Z^1$ represents an oxygen atom, are excluded.

Particularly important classes of compounds of the formula shown in Figure I include those which exhibit one or more of the following features:-

(i) $R^1$ represents a methyl or, more especially, a 2-haloethyl group, in particular a 2-chloroethyl group;

(ii) $R^2$ represents a cyano or azidocarbonyl group or a group of the formulae II to IX wherein $R^9$ and $R^{10}$ each represents a hydrogen atom or a methyl, ethyl, phenyl, 2,4-difluorophenyl or benzyl group, $R^{11}$ represents a hydrogen atom, $R^{12}$ represents a 2-chloroethyl, ethoxycarbonylmethyl or 2,2-dimethylpropyl group and $R^{13}$ and $R^{14}$ each represents a methyl group;

(iii) one of $A^1$ and $A^2$ represents a nitrogen atom and the other represents a group $-CR^3=$;

(iv) $R^3$ represents a hydrogen atom;

(v) $A^2$ represents a nitrogen atom;

(vi) $Z^1$ represents an oxygen atom; and/or

(vii) $Z^2$ represents an oxygen atom; other symbols being as hereinbefore defined.

Important individual compounds of the general formula shown in Figure I include the following:-

8-benzyloxycarbonyl-3-methyl-[3H]-imidazo[5,1-d]-1,2,3,5-tetrazin-4-one, A
8-carboxy-3-methyl-[3H ]-imidazo[5,1-d]-1,2,3,5-tetrazin-4-one, B
8-benzyloxycarbonyl-3-(2-chloroethyl)-[3H]-imidazo[5,1-d]-1,2,3,5-tetrazin-4-one, C
8-carboxy-3-(2-chloroethyl)-[3H]-imidazo[5,1-d]-1,2,3,5-tetrazin-4-one, D
3-(2-chloroethyl)-8-(N -methoxycarbamoyl)-[3H]-imidazo[5,1-d]-1,2,3,5-tetrazin-4-one, E
8-(N-benzyloxycarbamoyl)-3-(2-chloroethyl)-[3H]-imidazo[5,1-d]-1,2,3,5-tetrazin-4-one, F
8-(N-hydroxycarbamoyl)-3-(2-chloroethyl)-[3H]-imidazo[5,1-d]-1,2,3,5-tetrazin-4-one, G
3-(2-chloroethyl)-8-[N -(2-chloroethyl)carbamoyl]-[3H]-imidazo[5,1-d]-1,2,3,5-tetrazin-4-one, K
3-(2-chloroethyl)-8-phenylaminocarbamoyl-[3H]-imidazo[5,1-d]-1,2,3,5-tetrazin-4-one, N
3-(2-chloroethyl)-8-(2,4-difluorophenylamino carbamoyl)-[3H]-imidazo[5,1-d]-1,2,3,5-tetrazin-4-one, O
3-(2-chloroethyl)-8-methoxycarbonylaminocarbamoyl-[3H]-imidazo[5,1-d]-1,2,3,5-tetrazin-4-one, P
3-(2-chloroethyl)-8-diazoacetyl-[3H]-imidazo[5,1-d]-1,2,3,5-tetrazin-4-one, Q
1-[3-(2-chloroethyl)-[3H]-4-oxoimidazo[5,1-d]-1,2,3,5-tetrazine-8-carbonyl]-4-phenylsemicarbazide, R
8-carboxy-3-methyl-[3H]-imidazo[5,1-d]-1,2,3,5-tetrazin-4-one, S
8-[N-(ethoxycarbonylmethyl)carbamoyl]-3-(2-chloroethyl)-[3H]-imidazo[5,1-d]-1,2,3,5-tetrazin-4-one, T
S,S-dimethyl-N-[3-(2-chloroethyl)-[3H]-4-oxoimidazo[5,1-d]-1,2,3,5-tetrazin-8-ylcarbonyl]sulphoximide, U
8-azidocarbonyl-3-(2-chloroethyl)-[3H]-imidazo[5,1-d]-1,2,3,5-tetrazin-4-one, V
8-(2,2-dimethylpropylcarbamoyl-3-(2-chloroethyl)-[3H]-imidazo[5,1-d]-1,2,3,5-tetrazin-4-one, and W
8-ethylthiocarbonyl-3-(2-chloroethyl)-[3H]-imidazo[5,1-d]-1,2,3,5-tetrazin-4-one. AA

The letters A etc. are allocated to the compounds for easy reference later in the specification.

Compounds of particular importance include compounds D, E, G, K, N and Q.

The said tetrazine derivatives of the formula shown in Figure I possess valuable antineoplastic activity, and they also possess valuable immunomodulatory activity.

The tetrazine derivatives of the general formula shown in Figure I have proved particularly active in mice at daily doses between 0.2 and 320mg/kg animal body weight, administered intraperitoneally, against TLX5(S) lymphoma according to the procedure of Gescher et al., Biochem. Pharmacol. (1981), 30, 89, and ADJ/PC6A and M5076 (reticulum cell sarcoma). Against leukaemia L1210, grafted intraperitoneally, intracerebrally and intravenously, and P388, according to the procedure described in "Methods of Development of New Anticancer Drugs" (NCI Monograph 45, March 1977, pages 147-149, National Cancer Institute, Bethesda, United States), the compounds were active both intraperitoneally and orally at doses of between 1 and 320mg/kg animal body weight. Inhibition of both primary tumour and metastasis was obtained against

the Lewis lung carcinoma by similar dosage regimes. Against the B16 melanoma and C38 tumour in mice (NCI Monograph 45, op. cit.) the compounds were active intraperitoneally at doses of between 6.25 and 40 mg/kg animal body weight. Against colon carcinoma $C_{26}$ in mice, grafted subcutaneously, the compounds were active orally at doses of between 2 and 40mg/kg animal body weight.

According to a further feature of the invention there are provided acid halides of the general formula shown in Figure X, wherein $A^1$, $A^2$, $R^1$ and $Z^1$ are as hereinbefore defined and $X^1$ represents a halogen, e.g. chlorine, atom, which are useful as intermediates in the preparation of compounds of the formula shown in Figure I and also in the preparation of compounds described in the specifications of British Patents Nos. 2104522B and 2125402B, which also possess antineoplastic and immunomodulatory activity and the said West German Patent Application No. 2932305.

The compounds of the general formula shown in Figure I may be prepared by the application or adaptation of methods known per se.

For example, the compounds of the general formula shown in Figure I, especially those wherein $R^2$ represents a cyano group or an optionally substituted alkoxycarbonyl group, may be prepared by the reaction of a compound of the general formula shown in Figure XI of the drawings [wherein $A^1$ and $A^2$ are as hereinbefore defined and $R^{15}$ represents a group within the above definition of $R^2$, more especially a cyano group or an optionally substituted alkoxycarbonyl group] with a compound of the general formula:-

$R^1NCZ^1$ XII

wherein $R^1$ and $Z^1$ are as hereinbefore defined, and for the novel compounds of the general formula shown in Figure I, as hereinbefore defined, this constitutes a feature of the invention.

The reaction may be effected in the absence or presence of an anhydrous organic solvent, for example a chlorinated alkane, e.g. dichloromethane, or ethyl acetate, acetonitrile, N-methylpyrrolidin-2-one or hexamethylphosphoramide, at a temperature between 0° and 120°C. The reaction may be continued for up to 30 days. Light should preferably be excluded from the reaction mixture.

According to a further feature of the present invention, compounds of the general formula shown in Figure I wherein $R^2$ represents a carboxy or hydroxycarbamoyl group, $R^1$, $A^1$, $A^2$ and $Z^1$ being as hereinbefore defined, are prepared from corresponding compounds, within the general formula shown in Figure I, wherein $R^2$ represents a group of the formula $-COOR^{16}$ or $-CONHOR^{16}$ (wherein $R^{16}$ represents an optionally substituted benzyl group) by the application or adaptation of methods known per se for the replacement of optionally substituted benzyl groups by hydrogen atoms. Suitable reaction conditions include, for example, catalytic hydrogenation (using a catalyst such as palladium on charcoal and in a solvent such as ethyl acetate or dimethylformamide); or when $R^{16}$ represents a substituted benzyl group in which the substituent or at least one of the substituents carried by the benzyl group is an alkoxy (e.g. methoxy) group in the o-or p -position, preferably by reaction with trifluoroacetic acid, preferably in the presence of anisole, and usually at or near room temperature.

According to a further feature of the present invention, compounds of the general formula shown in Figure I wherein $R^2$ represents a carboxy group are prepared from compounds of the general formula shown in Figure XIII (wherein $A^1$, $A^2$, $R^1$ and $Z^1$ are as hereinbefore defined).

In one variant of this process the compound of the formula shown in Figure XIII is reacted with an aqueous solution of an alkali metal hypohalite, e.g. sodium hypochlorite, generally at between 0°C and room temperature, followed by acidification, e.g. with concentrated hydrochloric acid.

In another variant of this process the compound of the formula shown in Figure XIII is reacted with an alkali metal nitrite, e.g. sodium nitrite, and concentrated sulphuric acid or trifluoroacetic acid in the presence of water, generally at between room temperature and 60°C, followed by treatment with water, e.g. in the form of crushed ice.

According to a further feature of the invention, novel compounds of the general formula shown in Figure I wherein $R^2$ is other than a cyano group ($A^1$, $A^2$, $R^1$, $R^2$ and $Z^1$ being otherwise as hereinbefore defined) are prepared by the reaction of compounds of the general formula shown in Figure X ($A^1$, $A^2$, $R^1$, $X^1$ and $Z^1$ being as hereinbefore defined) with the appropriate compounds of the formulae XIV to XXII:-

$MN_3$ XIV

$R^{17}Z^2R^9$ XV

$NH_2OR^9$ XVI

$NH_2NR^9R^{10}$ XVII

$NH_2NHCOOR^9$ XVIII

$NH_2NHCONHR^9$ XIX

$CHR^{11} = N_2$ XX

$NH_2R^{12}$ XXI

$HN=S(O)R^{13}R^{14}$ XXII

wherein $R^9$, $R^{10}$, $R^{11}$, $R^{12}$, $R^{13}$, $R^{14}$ and $Z^2$ are as hereinbefore defined, M represents an alkali metal, e.g. sodium, atom, and $R^{17}$ represents a hydrogen atom or an alkali metal, e.g. sodium, atom.

Typically the reaction is carried out in an inert organic solvent medium, e.g. tetrahydrofuran, acetone or dichloromethane, optionally containing some water, at or below room temperature, optionally in the presence of an acid-binding agent, for example a tertiary amine, e.g. triethylamine, or pyridine.

The aforementioned salts of certain compounds of the formula shown in Figure I are prepared by the application of adaptation of methods known per se, for example by reaction of the parent compound of the formula shown in Figure I with an alkali metal hydroxide, carbonate or, preferably, bicarbonate, in an aqueous or aqueous-organic medium, or an alkali metal hydride in a suitable solvent, e.g. dimethylformamide, followed by isolation of the salt by methods known per se.

When a mixture of products is obtained in any of the abovementioned processes they may be separated by the application or adaptation of methods known per se, e.g. chromatography.

According to a further feature of the invention, compounds of the general formula shown in Figure X are prepared from compounds of the general formula shown in Figure I wherein $R^2$ represents a carboxy group, $A^1$, $A^2$, $R^1$ and $Z^1$ being as hereinbefore defined, by the application or adaptation of known methods for the preparation of acid halides from carboxylic acids, for example by reaction with the appropriate phosphorus halide. When $X^1$ represents a chlorine atom, a particularly suitable method is the reaction with thionyl chloride.

Compounds of the general formula shown in Figure XI may be prepared by the application or adaptation of methods known per se, for example methods described by Shealy et al., J. Org. Chem. (1961), 26, 2396, and Cheng et al., J. Pharm. Sci. (1968), 57, 1044, and methods described hereinafter in the Reference Examples.

Compounds of the general formula shown in Figure XIII may be prepared by the application or adaptation of methods known per se, for example methods described in the said specifications of British Patents Nos. 2104522B and 2125402B.

By the term 'methods known per se' as used in the present specification is meant methods heretofore used or described in the literature.

According to a further feature of the invention, there is provided a method for the preparation of compounds described in the specifications of British Patents Nos. 2104522B and 2125402B carrying a carbamoyl, monosubstituted carbamoyl or disubstituted carbamoyl group in the 8-position and compounds described in the specification of West German Patent Application No. 2932305 carrying an alkoxycarbonyl group in the 8-position by the reaction of compounds of the general formula shown in Figure X with ammonia or the appropriate amine, or the appropriate alkanol, in conditions similar to those described in the present specification for the reaction of compounds of the general formula shown in Figure X with compounds of formula XIV to XXII that is to say, as a feature of the present invention, compounds of the general formula shown in Figure XXIII, wherein $R^{18}$ represents a cycloalkyl group, or a straight-or branched-chain alkyl, alkenyl or alkynyl group containing up to 6 carbon atoms, each such alkyl, alkenyl or alkynyl group being unsubstituted or substituted by from one to three substituents selected from halogen (i.e. bromine, iodine or, preferably, chlorine or fluorine) atoms, straight-or branched-chain alkoxy, alkylthio, alkylsulphinyl and alkylsulphonyl groups each containing up to 4 carbon atoms, and optionally substituted phenyl groups, $A^3$ represents a nitrogen atom or a group $-CR^{20}=$ wherein $R^{20}$ represents a hydrogen atom or a substituent $R^{21}$ wherein $R^{21}$ represents a halogen atom, or a straight-or branched-chain alkyl or alkenyl group, containing up to 6 carbon atoms, which may carry up to 3 substituents selected from halogen atoms, optionally substituted phenyl groups, and straight-or branched-chain alkoxy, alkylthio and alkylsulphonyl groups containing up to 3 carbon atoms, or $R^{21}$ represents a cycloalkyl, cyano, nitro or optionally substituted phenoxy group or a group of the formula $-COR^{22}$ (wherein $R^{22}$ represents an alkyl or alkoxy group of up to 4 carbon atoms or an optionally substituted phenyl group), or $R^{21}$ represents a group of the formula:-

$-S(O)_nR^{23}$, $-SO_2NR^{24}R^{25}$ or $-CONR^{24}R^{25}$

(wherein n represents 0 or 2, $R^{23}$ represents a straight-or branched-chain alkyl or alkenyl group, containing up to 4 carbon atoms, which may carry an optionally substituted phenyl substituent, a cycloalkyl group or an optionally substituted phenyl group, $R^{24}$ and $R^{25}$, which may be the same or different, each represents a straight-or branched-chain alkyl or alkenyl group, containing up to 4 carbon atoms, which may carry an optionally substituted phenyl substituent, or a cycloalkyl group or an optionally substituted phenyl group or the group $-NR^{24}R^{25}$ represents a heterocyclic group, $A^4$ represents a nitrogen atom or, when $A^3$ represents a nitrogen atom, $A^4$ represents a nitrogen atom or a group $-CR^3=$ wherein $R^3$ is as hereinbefore defined, $Z^1$

represents an oxygen or sulphur atom, and $R^2$ represents a group of the formula:-
$-CONR^{24}R^{25}$
(wherein $R^{24}$ and $R^{25}$, which may be the same or different, each represents a straight-or branched-chain alkyl or alkenyl group, containing up to 4 carbon atoms, which may carry an optionally substituted phenyl substituent, or a cycloalkyl group or an optionally substituted phenyl group or the group $-NR^{24}R^{25}$ represents a heterocyclic group) are prepared by the reaction of compounds of the general formula shown in Figure XXIV ($A^3$, $A^4$, $R^{18}$, $X^1$ and $Z^1$ being as hereinbefore defined) with a compound of the general formula:-
$HNR^{24}R^{25}$ XXV
wherein $R^{24}$ and $R^{25}$ are as hereinbefore defined.

Typically the reaction is carried out in an inert organic solvent medium, e.g. tetrahydrofuran, acetone or dichloromethane, at or below room temperature.

As a feature of the present invention, compounds of the general formula shown in Figure XXVI, wherein $R^{26}$ represents a cycloalkyl group, or a straight-or branched-chain alkyl group containing up to 6 carbon atoms, optionally substituted by halogen or alkoxy containing up to 3 carbon atoms, or represents a methyl or ethyl group substituted by a phenyl group, $R^{27}$ represents an alkoxycarbonyl group containing up to 5 carbon atoms, $A^5$ represents a nitrogen atom or a group $-CR^{28}=$ wherein $R^{29}$ represents a hydrogen atom or a substituent $R^{29}$ wherein $R^{29}$ represents an alkyl, cycloalkyl or benzyl group, $A^6$ represents a nitrogen atom or a group $-CR^{28}=$ wherein $R^{28}$ represents a hydrogen atom or a substituent $R^{29}$ wherein $R^{29}$ represents an alkyl, cycloalkyl or benzyl group, are prepared by the reaction of compounds of the general formula shown in Figure XXVII ($A^5$, $A^6$, $R^{26}$ and $X^1$ being as hereinbefore defined) with a compound of the general formula:-
$R^{30}OH$ XXVIII
wherein $R^{30}$ represents an alkyl group containing up to 4 carbon atoms.

Typically the reaction is carried out without any extra organic solvents, at or below room temperature.

For example, this method is especially suitable for the preparation of the following compounds:-
3-(2-chloroethyl)-8-ethoxycarbonyl-[3H ]-imidazo[5,1-d]-1,2,3,5-tetrazin-4-one, H
8-ethoxycarbonyl-3-methyl-[3H]-pyrazolo[5,1-d]-1,2,3,5-tetrazin-4-one, I
3-(2-chloroethyl)-8-ethoxycarbonyl-[3H]-pyrazolo[5,1-d]-1,2,3,5-tetrazin-4-one, J
8-dimethylcarbamoyl-3-methyl-[3H]-imidazo [5,1-d]-1,2,3,5-tetrazin-4-one, X
8-tert-butylcarbamoyl-3-(2-chloroethyl)-[3H]-imidazo[5,1-d]-1,2,3,5-tetrazin-4-one, Y
8-methoxycarbonyl-3-(2-chloroethyl)-[3H]-imidazo[5,1-d]-1,2,3,5-tetrazin-4-one, Z
8-propoxycarbonyl-3-(2-chloroethyl)-[3H]-imidazo[5,1-d]-1,2,3,5-tetrazin-4-one, AB
8-isopropoxycarbonyl-3-(2-chloroethyl)-[3H]-imidazo[5,1-d]-1,2,3,5-tatrazin-4-one, AC
8-propylcarbamoyl-3-(2-chloroethyl)-[3H]-imidazo[5,1-d]-1,2,3,5-tetrazin-4-one, AD
8-allylcarbamoyl-3-(2-chloroethyl)-[3H]-imidazo[5,1-d]-1,2,3,5-tetrazin-4-one, AE
8-isopropylcarbamoyl-3-(2-chloroethyl)-[3H]-imidazo[5,1-d]-1,2,3,5-tetrazin-4-one, AF
8-cyclopropylcarbamoyl-3-(2-chloroethyl)-[3H]-imidazo[5,1-d]-1,2,3,5-tetrazin-4-one, and AH
8-cyclohexylcarbamoyl-3-(2-chloroethyl)-[3H]-imidazo[5,1-d]-1,2,3,5-tetrazin-4-one, AI
which form a feature of the present invention, especially when prepared by the said process, and 8-ethoxycarbonyl-3-methyl-[3H]-pyrazolo[5,1-d]-1,2,3,5-tetrazin-4-one I
which also forms a feature of the present invention, when prepared by the said process, or for the preparation of a pharmaceutical product for the treatment of the human or animal body against carcinomas, melanomas, sarcomas, lymphomas and leukaemias, and for the treatment of organ grafts and skin grafts and for the treatment of immunological diseases, and
8-phenylcarbamoyl-3-(2-chloroethyl)-[3H]-imidazo[5,1-d]-1,2,3,5-tetrazin-4-one, AG
8-dimethylcarbamoyl-3-(2-chloroethyl)-[3H]-imidazo[5,1-d]-1,2,3,5-tetrazin-4-one, and AJ
8-methylcarbamoyl-3-(2-chloroethyl)-[3H]-imidazo[5,1-d]-1,2,3,5-tetrazin-4-one, AK
which form a feature of the present invention when prepared by the said process.

The compounds
3-(2-chloroethyl)-8-cyano-[3H]-imidazo[5,1-d]-1,2,3,5-tetrazin-4-one and L
8-cyano-3-methyl-[3H]-imidazo[5,1-d]-1,2,3,5-tetrazin-4-one M
also form a feature of the present invention, especially when used for the preparation of a pharmaceutical product for the treatment of the human or animal body against carcinomas, melanomas, sarcomas, lymphomas and leukaemias, and for the treatment of organ grafts and skin grafts and for the treatment of immunological diseases,

The following Examples illustrate the preparation of compounds of the general formula shown in Figures I, XXIII and XXVI according to the present invention, and the Reference Examples illustrate the preparation of intermediates.

EXAMPLE 1

Compound A

A solution of crude benzyl 5-diazoimidazole-4-carboxylate (4.73g; prepared as described in Reference Example 1) in ethyl acetate (134ml) was treated with methyl isocyanate (11.6g) and the mixture was left to stand in the dark at room temperature overnight. The solution was then evaporated at 30°C/10mmHg. The residual solid was purified by medium pressure liquid chromatography on silica gel, eluting with ethyl acetate, and the product was triturated with petroleum ether, to give 8-benzyloxycarbonyl-3-methyl-[3H]-imidazo[5,1-d]-1,2,3,5-tetrazin-4-one (1.91g), in the form of colourless crystals, m.p. 171-173°C (with decomposition) [Elemental analysis: C,54.5;H,3.76;N,24.4%; calculated: C,54.7;H,3.89;N,24.6%].

EXAMPLE 2

Compound B

A shaking solution of 8-benzyloxycarbonyl-3-methyl-[3H]-imidazo[5,1-d]-1,2,3,5-tetrazin-4-one (0.4g; prepared as hereinbefore described in Example 1) in dimethylformamide (8ml) was hydrogenated at room temperature and atmospheric pressure, using a catalyst of palladium on charcoal (5%; 40mg). After 10 minutes, hydrogen absorption (40.1ml) was complete. The mixture was filtered and the filtrate was evaporated, and the resulting solid was triturated with ethyl acetate (4ml), to give 8-carboxy-3-methyl-[3H]-imidazo[5,1-d]-1,2,3,5-tetrazin-4-one in the form of a dimethylformamide solvate (0.22g), a light brown solid, m.p. 146-148°C (with decomposition) [NMR (in DMSO-$d_6$): singlets at 8.5 and 3.7ppm; I.R. (KBr disc): 1760, 1700$cm^{-1}$].

The said solvate (0.15g) was dissolved in acetone, treated with charcoal, filtered and evaporated, to give 8-carboxy-3-methyl-[3 H]-imidazo[5,1-d]-1,2,3,5-tetrazin-4-one hydrate (0.09g) in the form of a colourless solid, m.p. 173-174°C (with decomposition) [Elemental analysis: C,34.7;H,3.31;N,32.4%; $C_6H_5N_5O_3$:$H_2O$ requires: C,33.8;H,3.31;N,32.9%; NMR (in DMSO-$d_6$): singlets at 8.7 and 3.8ppm; I.R. (KBr disc): 1760, 1685$cm^{-1}$].

EXAMPLE 3

Compound C

A solution of crude benzyl 5-diazoimidazole-4-carboxylate (0.88g; prepared as described in Reference Example 1) in ethyl acetate (23ml) was treated with 2-chloroethyl isocyanate (3.68g) and was left to stand in the dark at room temperature overnight. The solution was then evaporated at 35°C and pressures down to 0.1mmHg. The resulting solid residue was purified by medium pressure chromatography on silica gel, eluting with a mixture of petroleum ether (b.p. 60-80°C) and ethyl acetate (1:1 v/v) and the product was washed with petroleum ether (b.p. 40-60°C), to give crude 8-benzyloxycarbonyl-3-(2-chloroethyl)-[3 H]-imidazo[5,1-d]-1,2,3,5-tetrazin-4-one (0.43g), in the form of pale yellow crystals, m.p. 110-112°C. [NMR (in DMSO-$d_6$): singlets at 8.7 and 5.4ppm, multiplet at 7.3ppm, triplets at 4.6 and 4.0ppm; I.R. (KBr disc): 1745, 1725$cm^{-1}$]. [Elemental analysis: C,50.6;H,3.64;N,21.0%; calculated: C,50.4;H,3.63;N,21.0%].

EXAMPLE 4

Compound D

A shaking solution of crude 8-benzyloxycarbonyl-3-(2-chloroethyl)-[3H]-imidazo[5,1-d]-1,2,3,5-tetrazin-4-one (0.36g; prepared as hereinbefore described in Example 3) in ethyl acetate (7ml) was hydrogenated at room temperature and atmospheric pressure, using a catalyst of palladium on charcoal (5%; 36mg) for 90 minutes, during which time 24.4ml of hydrogen was absorbed. The mixture was then diluted with acetone

(7ml) and filtered, and the filtrate was evaporated. The resulting residue was washed with ethyl acetate (5ml), to give 8-carboxy-3-(2-chloroethyl)-[3H]-imidazo[5,1-d]-1,2,3,5-tetrazin-4-one (0.14g), in the form of a colourless solid, m.p. 145°C (with decomposition). [NMR (in DMSO-$d_6$): singlet at 8.7ppm, triplets at 4.6 and 4.0ppm; I.R. (KBr disc): 1755, 1710$cm^{-1}$].

EXAMPLE 5

Compound D

A stirred, ice-cooled suspension of 8-carbamoyl-3-(2-chloroethyl)-[3H]-imidazo[5,1-d]-1,2,3,5-tetrazin-4-one (2.0g; prepared as described in the specification of British Patent No. 2104522B) in water (30ml) was treated with aqueous sodium hypochlorite solution (10.5% w/v available chlorine), maintaining the reaction temperature at 10° to 15°C. Unreacted starting material (0.29g) was filtered off and the filtrate was acidified to pH 1 by treatment with concentrated hydrochloric acid. The resulting precipitate was collected and washed with water, to give 8-carboxy-3-(2-chloroethyl)-[3H]-imidazo[5,1-d]-1,2,3,5-tetrazin-4-one hydrate (0.79g), in the form of colourless crystals, m.p. 162°C (with decomposition). [Elemental analysis: C,32.4;H,2.97;Cl,13.7;N,26.8;$H_2O$,6.2%; $C_7H_6O_3ClN_5$:$H_2O$ requires: C,32.1;H,3.06;Cl,13.6;N,26.8;$H_2O$,6.9%].

EXAMPLE 6

Compound E

A stirred solution of 3-(2-chloroethyl)-[3H]-4-oxoimidazo[5,1-d]-1,2,3,5-tetrazine-8-carbonyl chloride (2.26g; prepared as described in Example 21) in dry tetrahydrofuran (12.5ml) was treated dropwise with O-methyl hydroxylamine (0.8g). The mixture was allowed to stand at room temperature overnight and then it was concentrated to dryness in vacuo. The residual solid was subjected to medium pressure chromatography on silica gel, eluting with ethyl acetate, to give 3-(2-chloroethyl)-8-(N-methoxycarbamoyl)-[3H]-imidazo[5,1-d]-1,2,3,5-tetrazin-4-one (1.89g), in the form of colourless crystals, m.p. 124-126°C (with decomposition). [Elemental analysis: C,34.5;H,3.24;Cl,12.2;N,30.9%; calculated: C,35.24;H,3.33;Cl,13.0;N,30.83%; I.R. (KBr disc): 3290, 1750, 1690$cm^{-1}$, NMR (in DMSO-$d_6$): singlets at 3.8 and 8.85ppm, triplets at 4.07ppm (J = 6Hz) and 4.70ppm (J = 6Hz)].

EXAMPLE 7

Compound F

A stirred solution of 3-(2-chloroethyl)-[3H]-4-oxoimidazo[5,1-d]-1,2,3,5-tetrazin-8-carbonyl chloride (2.26g; prepared as described in Example 21) in dry tetrahydrofuran (12.5ml) was treated with O-benzyl hydroxylamine hydrochloride (1.37g). The mixture was stirred and cooled in an ice bath while treating dropwise with triethylamine (1.7g), and was then stirred at 0°C for one hour then it was left to stand at room temperature overnight. The mixture was then evaporated to dryness in vacuo, to give a gum which was subjected to medium pressure chromatography on silica gel, eluting with a mixture of toluene and ethyl acetate (1:1 v/v). The product was recrystallised from a mixture of petroleum ether (b.p. 60-80°C) and ethyl acetate, to give 8-(N-benzyloxycarbamoyl)-3-(2-chloroethyl)-[3H]-imidazo[5,1-d]-1,2,3,5-tetrazin-4-one (0.57g), in the form of a colourless solid, m.p. 139-141°C (with decomposition). [Elemental analysis: C,49.4;H,3.84;Cl,9.9;N,23.9%; calculated: C,48.2; H,3.76;Cl,10.2;N,24.1%; I.R. (KBr disc): 3380, 3130, 1750 and 1690$cm^{-1}$; NMR (in DMSO-$d_6$):-singlets at 4.90 and 8.75ppm, broad singlet at 11.7ppm, triplets at 3.95ppm (J = 6Hz) and 4.60ppm (J = 6Hz), multiplet at 7.2-7.4ppm].

EXAMPLE 8

Compound G

A solution of 8-(N-benzyloxycarbamoyl)-3-(2-chloroethyl)-[3H]-imidazo[5,1-d]-1,2,3,5-tetrazin-4-one (0.8g; prepared as described in Example 7) in ethyl acetate (120ml) and dimethylformamide (55ml) was hydrogenated at room temperature and atmospheric pressure, using a catalyst of palladium on charcoal (5%). The mixture was filtered and evaporated to dryness and the resulting solid was triturated with ethyl acetate, to give 8-(N -hydroxycarbamoyl)-3-(2-chloroethyl)-[3H]-imidazo[5,1-d]-1,2,3,5-tetrazin-4-one (0.4g), in the form of a colourless solid, m.p. 139-141°C (with decomposition). [Elemental analysis:- C,32.5;H,2.53;Cl,13.3;N,32.1%; calculated: C,32.51;H,2.73;Cl,13.71;N,32.5%; I.R. (KBr disc): 3300, 3160, 1750 and 1660cm$^{-1}$; NMR (in DMSO-$d_6$): singlet at 8.85ppm, broad singlets exchangeable with $D_2O$ at 9.25 and 11.27ppm, triplets at 4.03ppm (J = 6Hz) and 4.65ppm (J = 6Hz)].

EXAMPLE 9

Compound H

A solution of ethyl 5-diazoimidazole-4-carboxylate (1.27g; prepared as described in Reference Example 2) and 2-chloroethyl isocyanate (5.1ml) in ethyl acetate (46ml) was stirred in the dark for 3 days. The solution was concentrated in vacuo and the residue was triturated with petroleum ether (b.p. 60-80°), to give 3-(2-chloroethyl)-8-ethoxycarbonyl-[3H]-imidazo[5,1-d]-1,2,3,5-tetrazin-4-one (0.75g), in the form of a colourless solid, m.p. 77-79°C [Elemental analysis: C,39.6;H,3.64;Cl,13.2; N,25.5%; calculated: C,39.8;H,3.71;Cl,13.1;N,25.8%].

EXAMPLE 10

Compound I

A solution of ethyl 3-diazopyrazole-4-carboxylate (0.77g; prepared as described in Reference Example 3) in methyl isocyanate (0.7g) was left to stand at room temperature overnight. The resulting gum was subjected to medium pressure chromatography on silica gel, eluting with a mixture of petroleum ether (b.p. 60-80°C) and ethyl acetate (2:1 v/v), to give 8-ethoxycarbonyl-3-methyl-[3 H]-pyrazolo[5,1-d]-1,2,3,5-tetrazin-4-one (0.36g), in the form of a white solid, m.p. 136-137°C [Elemental analysis: C,43.3;H,4.13;N,31.4%; calculated: 43.05;H,4.06;N,31.4%; I.R. (KBr disc): 1760, 1710cm$^{-1}$; NMR (in DMSO-$d_6$): singlets at 4.0 and 8.65ppm, triplet at 1.35ppm (J = 8Hz), quartet at 4.38ppm (J = 8Hz)].

EXAMPLE 11

Compound J

Ethyl 3-diazopyrazole-4-carboxylate (2g; prepared as described in Reference Example 3) was dissolved in 2-chloroethyl isocyanate (2ml), and the solution was left to stand at room temperature for 7 days. The solution was triturated with excess petroleum ether (b.p. 60-80°C) and the gum which precipitated was collected. This gum was dissolved in a minimum volume of dichloromethane and subjected to medium pressure chromatography on silica gel, eluting with a mixture of petroleum ether (b.p. 60-80°C) and ethyl acetate (2:1 v/v) to give 3-(2-chloroethyl)-8-ethoxycarbonyl-[3H]-pyrazolo[5,1-d]-1,2,3,5-tetrazin-4-one (1.5g), in the form of colourless crystals, m.p. 98-99°C. [Elemental analysis: C,39.9;H,3.64;Cl,13.0;N,25.8% calculated: C,39.8;H,3.7;Cl,13.05;N,25.8%; I.R. (KBr disc): 3000, 1770, 1735 and 1595cm$^{-1}$; NMR (in DMSO-$d_6$): singlet at 8.45ppm, triplets at 1.37ppm (J= 6Hz), 4.0ppm (J = 6Hz) and 4.68ppm (J = 6Hz), quartet at 4.3ppm (J = 6Hz)].

EXAMPLE 12

Compound K

A solution of 3-(2-chloroethyl)-[3H]-4-oxoimidazo[5,1-d]-1,2,3,5-tetrazine-8-carbonyl chloride (5.5g; prepared as described in Example 21) in dry tetrahydrofuran (100ml) was treated with 2-chloroethylamine hydrochloride (2.42g) in one portion, and then with triethylamine (4.24g). The mixture was stirred at room temperature overnight and it was then heated on the steam bath for 1 hour. The mixture was then evaporated to dryness in vacuo and subjected to medium pressure chromatography on silica gel, eluting with a mixture of petroleum ether (b.p. 60-80°C) and ethyl acetate (1:1 v/v). The product was recrystallised from ethyl acetate, to give 3-(2-chloroethyl)-8-[ N-(2-chloroethyl)carbamoyl]-[3H]-imidazo[5,1-d]-1,2,3,5-tetrazin-4-one (1.13g) in the form of colourless microneedles, m.p. 130-132°C (with decomposition). [Elemental analysis: C,35.1;H,3.22;N,27.4;Cl,23.01%; calculated: C,35.43;H,3.30;N,27.54;Cl,23.24%; I.R. (KBr disc): 3360, 1730 and 1655cm$^{-1}$; NMR (in DMSO-$d_6$): singlets at 8.2 and 8.5ppm, triplets at 4.05 and 4.75ppm, multiplet at 3.7-3.9ppm].

EXAMPLE 13

Compound L

A stirred solution of 4-cyano-5-diazoimidazole [0.85g; prepared by methods described by Shealey et al., J. Pharm. Sci., (1975), 64, 954] in ethyl acetate (43ml) was treated with 2-chloroethyl isocyanate (4.7ml) and the resulting solution was stirred at 25°C in the dark for 3 days. The solution was concentrated in vacuo and the residue was triturated with petroleum ether (b.p. 60-80°C). The resulting insoluble residue was subjected to medium pressure chromatography on silica gel, eluting with ethyl acetate, to give 3-(2-chloroethyl)-8-cyano-[3H]-imidazo[5,1-d]-1,2,3,5-tetrazin-4-one (1.1g), in the form of a colourless solid, m.p. 108-110°C. [I.R. (in chloroform): 1755cm$^{-1}$; NMR (in DMSO-$d_6$):-singlet at 9.0ppm, triplets centred at 4.05 and 4.75ppm].

EXAMPLE 14

Compound M

A stirred solution of 4-cyano-5-diazoimidazole (2.2g) in ethyl acetate (110ml) was treated with methyl isocyanate (5.25g) and the resulting solution was stirred at room temperature for 4 days. A further portion of methyl isocyanate (5ml) was then added, and the solution was stirred at 40°C for a further period of 2 days. The dark solution was concentrated in vacuo and the residue was subjected to medium pressure chromatography on silica gel, eluting with a mixture of petroleum ether (b.p. 60-80°C) and ethyl acetate (1:1 v/v), to give 8-cyano-3-methyl-[3H ]-imidazo[5,1-d]-1,2,3,5-tetrazin-4-one (0.85g), in the form of a pinkish-white solid, m.p. 185-188°C [Elemental analysis: C,40.8;H,2.22;N,48.0%; calculated: C,40.91;H,2.29;N,47.72%].

EXAMPLE 15

Compound D

A stirred solution of 8-carbamoyl-3-(2-chloroethyl)-[3H]-imidazo[5,1-d]-1,2,3,5-tetrazin-4-one (1.0g; prepared as described in the specification of British Patent No. 2104522B) in concentrated sulphuric acid (8.0ml) at room temperature was treated with sodium nitrite (0.8g) and then with water (5 drops). The mixture was heated at 35°C for 90 minutes and then it was poured onto crushed ice. The resulting white solid was filtered off, to give 8-carboxy-3-(2-chloroethyl)-[3H]-imidazo[5,1-d]-1,2,3,5-tetrazin-4-one (0.85g), m.p.160-162°C (with decomposition).

EXAMPLE 16

Compound N

3-(2-Chloroethyl)-[3H]-imidazo[5,1-d]-1,2,3,5-tetrazine-8-carbonyl chloride (1.54g; prepared as described in Example 21) in tetrahydrofuran (5ml) was treated dropwise with phenylhydrazine (0.81g) in pyridine (3.5ml). The mixture was stirred at room temperature for 10 minutes and then it was poured into ice-cold dilute hydrochloric acid (100ml; 2.2N) and stirred. The resulting precipitate was recrystallised from aqueous acetone, to give 3-(2-chloroethyl)-8-phenylaminocarbamoyl-[3H]-imidazo[5,1-d]-1,2,3,5-tetrazin-4-one (0.55g) in the form of a yellow solid, m.p.140°C. [Elemental analysis: C,47.1;H,3.63;N,29.2%; calculated C,46.79;H,3.62; N,29.38%; I.R. (KBr disc): 3340, 3300, 1750, 1680, 1590, 1560, 1500, 1450cm$^{-1}$; NMR (in DMSO-$d_6$): singlets at 7.9, 8.85 and 10.4 ppm, triplets at 4.0 and 4.63, quartet at 7.0 ppm].

EXAMPLE 17

Compound O

By proceeding in a manner similar to that described hereinbefore in Example 16, but replacing the phenylhydrazine by the appropriate quantity of 2,4-difluorophenylhydrazine, there was prepared 3-(2-chloroethyl)-8-(2,4-difluorophenylaminocarbamoyl)-[3 H]-imidazo[5,1-d]-1,2,3,5-tetrazin-4-one in the form of a bright yellow solid [Elemental analysis: C,41.6;H,2.40;N,26.3%; calculated: C,42.2;H,2.72;N,26.5%; I.R. (KBr disc): 3320, 3180, 1740, 1650, 1500, 1450, 1250, 960cm$^{-1}$; NMR (in DMSO-$d_6$): singlets at 8.9 and 10.4 ppm, triplets at 4.0 and 4.6 ppm, broad band at 7.0 ppm].

EXAMPLE 18

Compound P

By proceeding in a manner similar to that described hereinbefore in Example 16, but replacing the phenylhydrazine by the appropriate quantity of methyl carbazate, there was prepared 3-(2-chloro ethyl)-8-methoxycarbonylaminocarbamoyl-[3 H]-imidazo[5,1-d]-1,2,3,5-tetrazin-4-one in the form of a white solid, m.p. 170°C [Elemental analysis: C,34.6;H,3.12;N,30.7%; calculated: C,34.24;H,3.19;N,31.06%; I.R. (KBr disc): 3280, 3240, 1730, 1680, 1560, 1450, 1240, 1220cm$^{-1}$; NMR (in DMSO-$d_6$): singlets at 3.6, 8.86, 9.2 and 10.26 ppm, triplets at 4.0 and 4.63 ppm].

EXAMPLE 19

Compound Q

3-(2-Chloroethyl)-[3 H]-4-oxoimidazo[5,1-d]-1,2,3,5-tetrazine-8-carbonyl chloride (1g; prepared as described in Example 21), cooled in an ice bath in the dark, was treated slowly with diazomethane (1g) in diethyl ether (75ml) and the reaction was allowed to continue in these conditions for 30 minutes. The resulting yellow solid was recrystallised from aqueous acetone, to give 3-(2-chloroethyl)-8-diazoacetyl-[3H]-imidazo[5,1-d]-1,2,3,5-tetrazin-4-one (0.72g) in the form of a buff solid, m.p. 90°C [Elemental analysis: C,34.1;H,2.9;N,34.8%, calculated: C,33.6;H,2,8;N,34.3%; I.R. (KBr disc): 3600, 3440, 3100, 3030, 2100, 1750, 1730, 1580, 1340, 1130 cm$^{-1}$; NMR (in DMSO-$d_6$): singlets at 6.76 and 8.8 ppm, triplets at 3.96 and 4.56 ppm].

EXAMPLE 20

Compound R

4-Phenylsemicarbazide (1.13g) in pyridine (3.5ml) and tetrahydrofuran (5ml) was treated dropwise with 3-(2-chloroethyl)-[3H]-4-oxoimidazo[5,1-d]-1,2,3,5-tetrazine-8-carbonyl chloride (1.53g; prepared as described in Example 21) in tetrahydro-furan (5ml). The mixture was stirred at room temperature for 15 minutes and then it was poured into dilute hydrochloric acid (100ml; 2.2N) and stirred. The resulting precipitate was recrystallised from aqueous acetone, to give 1-[3-(2-chloroethyl)-[3H]-4-oxoimidazo[5,1-d]-1,2,3,5-tetrazine-8-carbonyl]-4-phenylsemicarbazide (0.76g) in the form of a buff solid, m.p. 169°C, thought to be the monohydrate [Elemental analysis: C,42.6;H,37.8;N,28.4%; calculated for monohydrate: C,42.59;H,3.80;N,28.40%; I.R. (KBr disc): 3360, 3240, 3140, 1745, 1665, 1660, 1540, 1320, 1240, 900cm$^{-1}$; NMR (in DMSO-$d_6$): singlets at 8.26, 8.84 and 10.24 ppm, doublet at 7.48 ppm, triplets at 4.06, 4.68, 6.98 and 7.28 ppm].

EXAMPLE 21

A suspension of 8-carboxy-3-(2-chloroethyl)-[3H]-imidazo[5,1-d]-1,2,3,5-tetrazin-4-one hydrate (2.2g; prepared as described in Example 5) in thionyl chloride (50 ml) was heated at reflux until complete solution was achieved. The solution was then concentrated in vacuo, treated with toluene and concentrated in vacuo again, to give 3-(2-chloroethyl)-[3H]-4-oxoimidazo[5,1-d]-1,2,3,5-tetrazine-8-carbonyl chloride (2.5g), in the form of a yellow gum which was used directly in the next stage.

EXAMPLE 22

Compound D

8-Carbamoyl-3-(2-chloroethyl)-[3H]-imidazo[5,1-d]-1,2,3,5-tetrazin-4-one (10 g; prepared as described in the specification of British Patent No. 2104522B) was treated with concentrated sulphuric acid (50 ml) with stirring. When the solid had dissolved the solution was carefully treated with a solution of sodium nitrite (10 g) in water (25 ml) over 30 minutes, keeping the temperature below 60°C. The resulting yellow solution was stirred at between 35° and 40°C for 2.5 hours and then it was poured onto ice (300 g). The solid was filtered off, washed well with water and dried in vacuo, to give 8-carboxy-3-(2-chloroethyl)-[3H]-imidazo[5,1-d]-1,2,3,5-tetrazin-4-one (7.1 g). [I.R. (KBr disc) : 3500,1760, 1710, 1570, 1460 cm$^{-1}$; mass spectrum 243/245 ($M^+$)].

EXAMPLE 23

Compound D

A suspension of 8-carbamoyl-3-(2-chloroethyl)-(3H]-imidazo-[4,1-d]-1,2,3,5-tetrazin-4-one (5.0 g)in trifluoroacetic acid (20 ml) was treated dropwise with a solution of sodium nitrite (5.0 g) in water (10 ml). The mixture was stirred at 35°C for 2 hours. The yellow solution obtained was poured onto crushed ice to give a cream solid, which was filtered off, to give 8-carboxy-3-(2-chloroethyl)-[3H]-imidazo-[5,1-d]-1,2,3,5-tetrazin-4-one (4.77g).

EXAMPLE 24

Compound S

A stirred solution of 8-carbamoyl-3-methylimidazo-[5,1-d]-1,2,3,5-tetrazin-4-one (1.0 g) in concentrated sulphuric acid (8.0 ml) was treated with sodium nitrite (0.5 g) at room temperature. The reaction mixture was treated with water (five drops). It was stirred for 15 minutes at 35°C and then poured onto crushed ice, to give 8-carboxy-3-methyl-[3H]-imidazo-[5,1-d]-1,2,3,5-tetrazin-4-one (0.9 g) in the form of white micro-crystals, which were collected by filtration. [m.p. 178-180°C (from aqueous acetone). Elemental analysis: C, 35.9; H, 2.75; N, 35.5%; $C_6H_5N_5O_3$. 0.25 $H_2O$ requires: C, 36.08; H, 2.75; N, 35.1%; I.R. (KBr disc): 3640, 3350, 3160, 1745, 1670 $cm^{-1}$;U.V.(in 95%ethanol)325 nm; N.M.R. (in deuterated dimethylsulphoxide) : singlets at 8.62 and 3.90 p.p.m; mass spectrum 195 ($M^+$)].

EXAMPLE 25

Compound T

A solution of 3-(2-chloroethyl)-[3H]-4-oxoimidazo-[5,1-d]-1,2,3,5-tetrazine-8-carbonyl chloride (0.52 g) in dichloromethane (8 ml) was treated with a mixture of glycine ethyl ester hydrochloride (0.28 g) and triethylamine (0.28 g) in dichloromethane (10 ml). The mixture was stirred at ambient temperature for 1 hour, and then the dichloromethane solvent was removed by evaporation. The residue was treated with water and the resulting solution was stirred for 30 minutes. The precipitate was filtered off and recrystallised from ethyl acetate to give 8-[N-(ethoxycarbonylmethyl)carbamoyl]-3-(2-chloroethyl)-[3H]-imidazo-[5,1-d]-1,2,3,5-tetrazin-4-one in the form of a pink solid (0.28 g). [Elemental analysis: C, 39.7; H, 4.05; N, 24.3%; calculated C, 40.1; H, 3.99; N, 25.5%; I.R. (KBr disc): 3400, 1750, 1670, 1590, 1540, 1470 $cm^{-1}$; N.M.R. (in deuterated dimethylsulphoxide) : singlets at 8.0, 8.85, triplet at 4.65, multiplets at 1.2 and 4.05 p.p.m.].

EXAMPLE 26

Compounds U, V and W

A solution of 3-(2-chloroethyl)-[3H]-4-oxoimidazo-[5,1-d]-1,2,3,5-tetrazine-8-carbonyl chloride (1.5 g) in dichloromethane (5 ml) was treated dropwise with a solution of S,S-dimethylsulphoximide (0.53 g) and triethylamine (0.58 g) in dichloromethane (5 ml). The reaction flask was suspended in an ice/acetone bath and the reaction was allowed to proceed for 20 minutes. A precipitate formed which was filtered off and recrystallised from aqueous acetone, to give S,S-dimethyl-N-[3-(2-chloroethyl)-[3H]-4-oxoimidazo-[5,1-d]-1,2,3,5-tetrazin-8-ylcarbonyl]sulphoximide (1.42 g) in the form of a buff coloured solid. [Elemental analysis: C, 34.1; H, 3.38; N, 26.3%; calculated: C, 33.91; H, 3.48; N, 26.4%; I.R. (KBr disc) 3000, 2910, 1730, 1600, 1440, 1225, 1180 $cm^{-1}$; N.M.R. (in deuterated dimethylsulphoxide) : singlets at 3.5 and 8.7, triplets at 4.0 and 4.6 p.p.m.].

By proceeding in a similar manner but replacing the S,S-dimethylsulphoximide by the appropriate quantity of sodium azide, using aqueous acetone as the reaction medium and working at the ambient temperature, there was prepared 8-azidocarbonyl-3-(2-chloroethyl)-[3H]-imidazo-[5,1-d]-1,2,3,5-tetrazin-4-one in the form of a white solid. [Elemental analysis: C, 31.3; H, 1.72; N, 41.7%; Calculated: C, 31.3; H, 1.88; N, 41.7%); I.R. (KBr disc) 3150, 2175, 1760, 1720, 1580, 1480, 1210 $cm^{-1}$; N.M.R. (in deuterated dimethylsulphoxide) : singlet at 8.95, triplets at 4.05 and 4.65 p.p.m.].

By again proceeding in a similar manner but replacing the S,S-dimethylsulphoximide by the appropriate quantity of 2,2-dimethylpropylamine, using tetrahydrofuran as the reaction medium and working at the ambient temperature, there was prepared 8-(2,2-dimethylpropylcarbamoyl-3-(2-chloroethyl)-[3H]-imidazo-[5,1-d]-1,2,3,5-tetrazin-4-one in the form of a cream solid. [Elemental analysis: C, 45.7; H, 5.21; N, 26.8%; calculated: C, 46.08; H, 5.48; N, 26.87%; N.M.R. (in deuterated dimethylsulphoxide) : singlets at 0.9 and 8.83, triplets at 3.1, 4.0 and 4.6, broad at 8.13 p.p.m.].

EXAMPLE 27

By proceeding as hereinbefore described in Example 21, but with the inclusion of a trace of dimethylformamide in the reaction mixture, 3-(2-chloroethyl)-[3H]-4-oxoimidazo-[5,1-d]-1,2,3,5-tetrazine-8-carbonyl chloride was obtained in the form of a light brown crystalline solid, m.p. 69°C. [I.R. (KBr disc): 1730, 1520, 1450, 1320, 1230 $cm^{-1}$; mass spectrum 261/263 ($M^+$)].

EXAMPLE 28

By proceeding as hereinbefore in Example 27, but using the appropriate quantity of 8-carboxy-3-methyl-[3H]-imidazo-[5,1-d]-1,2,3,5-tetrazin-4-one (prepared as described in Example 24) as the starting material, there was prepared 3-methyl-[3H ]-oxoimidazo-[5,1-d]-1,2,3,5-tetrazine-8-carbonyl chloride, in the form of a yellow solid, m.p. 70-75°C. [I.R. (KBr disc) 1735 $cm^{-1}$; mass spectrum 213/215 ($M^+$)].

EXAMPLE 29

Compound X

A suspension of 3-methyl-[3 H]-oxoimidazo-[5,1-d]-1,2,3,5-tetrazine-8-carbonylchloride (0.4 g; prepared as described in Example 28) in dichloromethane (10 ml) was treated with a solution of dimethylamine in dichloromethane (extracted from 40% aqueous dimethylamine solution), dropwise with constant stirring, until the reaction mixture was slightly alkaline. The solution was stirred for a further 20 minutes. The dichloromethane was removed under reduced pressure and the remaining brown oil was triturated with a mixture of acetone and petroleum ether (b.p. 60-80°C) to give 8-dimethylcarbamoyl-3-methyl-[3H]-imidazo-[5,1-d]-1,2,3,5-tetrazin-4-one (0.3 g) in the form of a yellowish brown solid, m.p. 130-135°C (with decomposition). [I.R. (KBr disc) $1730^{-1}$; mass spectrum 222 ($M^+$)].

EXAMPLE 30

Compounds Y, AA, AD, AE, AF, AG, AH, AI, AJ and AK

A stirred solution of 3(2-chloroethyl)-[3H]-4-oxoimidazo-[5,1-d]-1,2,3,5-tetrazine-8-carbonyl chloride (2.5 g; prepared as described in Example 21) in dry tetrahydrofuran was treated with tert-butylamine (1.3 g) dropwise, cooling in an ice bath. Stirring was continued for 5 minutes at 10°C to 12°C and then for one hour at room temperature. The mixture was then concentrated in vacuo and the residue was subjected to flash chromatography on silica gel using ethyl acetate as the eluant, to give 8-tert-butylcarbamoyl-3-(2-chloroethyl)-[3H]-imidazo-[5,1-d]-1,2,3,5-tetrazin-4-one (2.2 g) in the form of colourless crystals, m.p. 126-128°C (with decomposition). [Elemental analysis: C, 44.4; H, 5.12; Cl, 12.0; N, 28.4%; calculated: C, 44.23; H, 5.06; Cl,11.87; N, 28.13%; I.R. (KBr disc) 3410, 3130, 1750 and 1675 $cm^{-1}$. N.M.R. (in deuterated dimethylsulphoxide) : singlets at 1.45, 8.9, broad singlet at 7.6 and triplets at 4.05 and 4.65 p.p.m.].

By proceeding in a similar manner, but replacing the tert-butylamine with the appropriate quantities of the reagents indicated, the following products were obtained.

Ethanethiol (without solvent; and recrystallising the product from aqueous acetone) gave 8-ethylthiocarbonyl-3-(2-chloroethyl)-[3H]-imidazo-[5,1-d]-1,2,3,5-tetrazin-4-one in the form of a white solid. [Elemental analysis: C, 37.4; H, 3.32; N, 24.2%; calculated C, 37.57; H, 3.50; N, 24.34%; I.R. (KBr disc): 1760, 1640, 1460, 1330, 1290, 1250 $cm^{-1}$; N.M.R. (in deuterated dimethylsulphoxide) : singlet at 8.95, triplets at 1.3, 4.05, 4.65, quartet at 3.05 p.p.m.].

Propylamine (in dichloromethane; and recrystallising the product from aqueous acetone) gave 8-propylcarbamoyl-3-(2-chloroethyl)-[3H]-imidazo-[5,1-d]-1,2,3,5-tetrazin-4-one in the form of a pale purple solid. [Elemental analysis: C, 42.1; H, 4.7; N, 29.6%; calculated: C, 42.19; H, 4.60; N, 29.52%; I.R. (KBr disc): 3450, 3150, 3000, 1760, 1650, 1590, 1470 $cm^{-1}$; N.M.R. (in deuterated dimethylsulphoxide) : singlets at 8.45, 8.85, triplets at 0.8, 4.0, 4.6, quartets at 1.5, 3.3 p.p.m.].

Allylamine (in dichloromethane; and recrystallising the product from aqueous acetone) gave 8-allylcarbamoyl-3-(2-chloroethyl)-[3H]-imidazo-[5,1-d]-1,2,3,5-tetrazin-4-one in the form of a pale purple solid. [Elemental analysis: C, 42.5; H, 3.79; N, 29.9%; calculated: 42.49; H, 3.92; N, 29.73%; I.R. (KBr disc): 3300, 1780, 1660, 1580, 1480, 1260 $cm^{-1}$; N.M.R. (in deuterated dimethylsulphoxide) : singlets at 5.95, 8.6, 8.8, triplets at 4.75, 5.25, quartet at 4.15 p.p.m.].

Isopropylamine (in dichloromethane; and recrystallising the product from aqueous acetone) gave 8-isopropylcarbamoyl-3-(2-chloroethyl)-[3H]-imidazo-[5,1-d]-1,2,3,5-tetrazin-4-one in the form of a cream solid. [Elemental analysis: C, 42.4; H, 4.52; N, 29.2%; calculated: C, 42.19; H, 4.60; N, 29.52%; I.R. (KBr disc): 3450, 3150, 1760, 1690, 1590, 1540 $cm^{-1}$; N.M.R. (in deuterated dimethylsulphoxide) : singlet at 8.9, doublets at 1.2, 8.15, triplet at 4.65, quartet at 4.1 p.p.m.].

Aniline (in dichloromethane; and recrystallising the product from aqueous acetone) gave 8-phenylcarbamoyl-3-(2-chloroethyl)-[3H]-imidazo-[5,1-d]-1,2,3,5-tetrazin-4-one in the form of a deep yellow solid. [Elemental analysis: C, 48.4; H, 3.29; N, 26.1%; calculated: C, 48.9; H, 3.48; N, 26.3%; I.R. (KBr disc): 3400, 3150, 1750, 1700, 1610, 1530, 1460 $cm^{-1}$; N.M.R. (in deuterated dimethylsulphoxide) : singlets at 9.0, 10.35, doublet at 8.85, triplets at 4.05, 4.65, quartet at 7.25 p.p.m.]

Cyclopropylamine (in dichloromethane; and recrystallising the product from aqueous acetone) gave 8-cyclopropylcarbamoyl-3-(2-chloroethyl)-[3H ]-imidazo-[5,1-d]-1,2,3,5-tetrazin-4-one monohydrate in the form of a white solid, m.p. 132°C. [Elemental analysis: C, 39.6; H, 4.21; N, 28.2%; calculated for the monohydrate:-C, 39. ; H, 4.36; N, 27.94%; I:R. (KBr disc): 3600, 3450, 3300, 1760, 1630, 1580, 1470, 1270 $cm^{-1}$; N.M.R. (in deuterated dimethylsulphoxide) : singlet at 8.8, doublet at 8.5, triplets at 4.1, 4.6 p.p.m.].

Cyclohexylamine [in pyridine; reaction time 10 minutes; reaction mixture poured into cold hydrochloric acid (2N) and recrystallising the precipitate from aqueous acetone] gave 8-cyclohexylcarbamoyl-3-(2-chloroethyl)-[3H]-imidazo-[5,1-d]-1,2,3,5-tetrazin-4-one in the form of a white solid, m.p. 130°C. [Elemental analysis: C, 48.3; H, 5.31; N, 25.8%; calculated: C, 48.08; H, 5.3; N, 25.88%; I.R. (KBr disc): 3400, 3110, 2940, 2850, 1750, 1660, 1570, 1500, 1450, 1250 $cm^{-1}$; N.M.R. (in deuterated dimethylsulphoxide) : singlet at 8.8, doublet at 8.1, triplets at 4.0, 4.6, broad band at 1.5 p.p.m..

Dimethylamine (40% aqueous solution in tetrahydrofuran; reaction time 20 minutes; and recrystallising the product from aqueous acetone) gave 8-dimethylcarbamoyl-3-(2-chloroethyl)-[3H]-imidazo-[5,1-d]-1,2,3,5-tetrazin-4-one in the form of a white solid. [Elemental analysis: C, 39.5; H, 4.00; N, 30.9%; calculated: C, 39.94; H, 4.09; N, 31.05%; I.R. (KBr disc): 3050, 2920, 1720, 1610, 1450, 1350, 1225 $cm^{-1}$; N.M.R. (in deuterated dimethylsulphoxide) : singlet at 8.0, doublets at 1.0, 7.4, triplets at 3.63, 4.2 p.p.m.].

Methylamine (40% aqueous solution in tetrahydrofuran; reaction time 30 minutes; and recrystallising the product from aqueous acetone) gave 8-methylcarbamoyl-3-(2-chloroethyl)-[3H]-imidazo-[5,1-d]-1,2,3,5-tetrazin-4-one monohydrate in the form of a white solid. [Elemental analysis: C, 35.0; H, 3.84; N, 30.8%; calculated for the monohydrate: C, 34.98; H, 4.04; N, 30.60%; I.R. (KBr disc): 3540, 3450, 3280, 1750, 1640, 1570, 1460, 1260 $cm^{-1}$; N.M.R. (in deuterated dimethylsulphoxide) : singlets at 8.8, doublets at 2.8, triplets at 4.0, 4.6, broad band at 8.4 p.p.m.].

EXAMPLE 31

Compounds Z, H, AB and AC

By proceeding in a manner similar to that hereinbefore described in Example 30, but replacing the tert-butylamine with the appropriate quantities of the reagents indicated, the following products were obtained.

Methanol (without solvent; and recrystallising the product from aqueous acetone) gave 8-methoxycarbonyl-3-(2-chloroethyl)-[3H]-imidazo-[5,1-d]-1,2,3,5-tetrazin-4-one in the form of a white solid. [Elemental analysis: C, 37.1; H, 3.0; N, 27.0%; calculated: C, 37.3; H, 3.13; N, 27.2%; I.R. (KBr disc): 1780, 1750, 1480, 1370, 1280, 1190 $cm^{-1}$; N.M.R. (in deuterated dimethylsulphoxide) : singlet at 8.9, triplet at 4.65, multiplet at 4.0 p.p.m.].

Ethanol (without solvent; and recrystallising the product from aqueous acetone) gave 8-ethoxycarbonyl-3-(2-chloroethyl)-[3H]-imidazo-[5,1-d]-1,2,3,5-tetrazin-4-one in the form of a white solid. [Elemental analysis: C, 39.6; H, 3.65; N, 25.9%; calculated: C, 39.79; H, 3.71; N, 25.78%; I.R. (KBr disc): 1780, 1740, 1480, 1260, 1210 $cm^{-1}$; N.M.R. (in deuterated dimethylsulphoxide) : singlet at 8.9, triplets at 1.35, 4.0, 4.65, quartet at 4.35 p.p.m.].

Propanol (without solvent; and recrystallising the product from aqueous acetone) gave 8-propoxycarbonyl-3-(2-chloroethyl)-[3H]-imidazo-[5,1-d]-1,2,3,5-tetrazin-4-one in the form of a fine white powder. [Elemental analysis: C, 42.1; H, 4.15; N, 24.6%; calculated: C, 42.04; H, 4.23; N, 24.51%; I.R. (KBr disc): 1760, 1730, 1580, 1480, 1350, 1280 $cm^{-1}$; N.M.R. (in deuterated dimethylsulphoxide) : singlet at 8.9, triplets at 1.0, 4.05, 4.3, 4.65, quartet at 1.7 p.p.m.].

Isopropanol (without solvent; and recrystallising the product from aqueous acetone) gave 8-isopropoxycarbonyl-3-(2-chloroethyl)-[3H]-imidazo-[5,1-d]-1,2,3,5-tetrazin-4-one in the form of a white solid. [Elemental analysis: C, 42.2; H, 4.18 N, 24.6%; Cl, 12.3%; calculated: C, 42.04; H, 4.23; N, 24.51;Cl, 12.4%; I.R. (KBr disc): 1760, 1740, 1580, 1340, 1280 $cm^{-1}$; N.M.R. (in deuterated dimethylsulphoxide) : singlet at 8.85, doublet at 1.35, triplets at 4.0, 4.56, quartet at 5.2 p.p.m.].

REFERENCE EXAMPLE 1

(i) An intimate mixture of 5-nitroimidazole-4-carboxylic acid (2.0g) and phosphorus pentachloride (2.67g) was stirred and heated in an oil bath at 120°C for 1 hour. The resulting yellow slurry was evaporated at 60°C/0.1mmHg for 30 minutes, to give 1,6-dinitro-5H,10H-diimidazo[1,5-a:1',5'-d]pyrazine-5,10-dione (1.90g) in the form of a yellow solid, m.p. 249-251°C (with decomposition). [I.R. (KBr disc) 1750cm$^{-1}$; m/e 278 ($M^+$)].
Windaus, Ber., 1923, 56, 684 and Gireva, Chem. Abs. 59, 1622e, using the same method, describe their products as "5-nitroimidazole-4-carbonyl chloride".

(ii) A stirred solution of triethylamine (9.1ml) in benzyl alcohol (91ml) was treated portionwise with 1,6-dinitro-5H,10H -diimidazo[1,5-a:1',5'-d]pyrazine-5,10-dione (9.07g) during 5 minutes, while the temperature rose to 50°C. The temperature was maintained at 60°C for 15 minutes to obtain complete solution. The solution was then cooled, acidified with saturated ethereal hydrogen chloride solution (45ml), and diluted with diethyl ether (400ml). The resulting colourless precipitate was filtered off, washed with water, and recrystallised from ethanol, to give benzyl 5-nitroimidazole-4-carboxylate (9.95g), in the form of pale yellow crystals, m.p. 205-207°C (with decomposition) [Elemental analysis: C,53.7;H,3.62;N,17.0%; calculated: C,53.4;H,3.67;N,17.0%].

(iii) A shaking solution of benzyl 5-nitroimidazole-4-carboxylate (1.0g) in methanol (18ml) and dimethylformamide (12ml) was hydrogenated at room temperature and atmospheric pressure, using a catalyst of platinum oxide (0.1g). After 20 minutes hydrogen absorption (260ml) was complete. The mixture was treated with charcoal and filtered with the aid of diatomaceous earth. The filtrate was evaporated, to give crude benzyl 5-aminoimidazole-4-carboxylate (0.97g), in the form of a brown oil. [NMR (in DMSO-$d_6$): singlets at 7.2 and 5.2ppm, multiplet at 7.3ppm].

(iv) Crude benzyl 5-aminoimidazole-4-carboxylate (4.89g; prepared as hereinbefore described) was dissolved in dilute hydrochloric acid (1N;54ml), and the solution was treated with charcoal and filtered. The yellow filtrate was added dropwise to a stirred solution of sodium nitrite (2.04g) in water (15ml) during 5 minutes at between 5° and 10°C. The resulting gummy suspension was extracted with ethyl acetate (3 x 125ml), the extract was dried over magnesium sulphate and evaporated at 35°C/10mmHg, to give crude benzyl 5-diazoimidazole-4-carboxylate (4.73g), in the form of a yellow oil. [I.R. (film): 2200cm$^{-1}$].

REFERENCE EXAMPLE 2

A solution of ethyl 5-aminoimidazole-4-carboxylate [1.1g; prepared by methods described by Davis et al., J. Chem. Soc., (1949) 1071] in hydrochloric acid (1N; 27.1ml) was treated dropwise with a solution of sodium nitrite (0.55g) in water (5ml), maintaining the temperature at 0°C. The resulting solution was left to stand at 0°C for 90 minutes, and then it was extracted with chloroform (4 x 25ml). The combined extracts were dried over magnesium sulphate and concentrated in vacuo, to give ethyl 5-diazoimidazole-4-carboxylate (1.27g) in the form of a solid.

REFERENCE EXAMPLE 3

A solution of ethyl 3-aminopyrazole-4-carboxylate [0.78g; prepared by methods described by Schmidt et al., Helv. Chim. Acta, (1956), 39 986] in water (10ml) and concentrated hydrochloric acid (5ml) was cooled to 0°C and treated dropwise with a solution of sodium nitrite (0.35g) in water (2ml). The mixture was stirred for 30 minutes at 0°C and then it was poured into chloroform (60ml). The mixture was treated gradually with solid sodium carbonate until the aqueous phase was at a pH between 7 and 8. The layers were separated and the aqueous phase was extracted four times with methylene chloride. The combined extracts were dried over sodium sulphate and evaporated to dryness, to give ethyl 3-diazopyrazole-4-carboxylate (0.77g), in the form of pale yellow crystalline needles [I.R. (in chloroform): 2180cm$^{-1}$; NMR (in DMSO-$d_6$): singlet at 8.12ppm, triplet at 1.4ppm (J = 7Hz), quartet at 4.36ppm (J = 6Hz)].

The present invention includes within its scope pharmaceutical compositions which comprise, as active ingredient, at least one compound of the general formula shown in Figure I, together with a pharmaceutical carrier or coating. In clinical practice the compounds of the general formula shown in Figure I will normally be administered orally, rectally, parenterally, for example intraperitoneally or intravenously, e.g. by infusion, or vaginally.

Methods of presentation of pharmaceutically active compounds are well known in the art and a suitable vehicle may be determined by the physician or pharmacist, depending upon such factors as the effect sought, the size, age, sex and condition of the patient and on the properties of the active compound. The compositions may also contain, as is usual in the art, such materials as solid or liquid diluents, wetting agents, preservatives, flavouring and colouring agents and the like.

Solid compositions for oral administration include compressed tablets, pills, dispersible powders, and granules. In such solid compositions one or more of the active compounds is, or are, admixed with at least one inert diluent such as calcium carbonate, potato starch, alginic acid, or lactose. The compositions may also comprise, as is normal practice, additional substances other than inert diluents, e.g. lubricating agents, such as magnesium stearate. Liquid compositions for oral administration include pharmaceutically-acceptable emulsions, solutions, suspensions, syrups and elixirs containing inert diluents commonly used in the art, such as water and liquid paraffin. Besides inert diluents such compositions may also comprise adjuvants, such as wetting and suspending agents, e.g. polyvinylpyrrolidone, and sweetening, flavouring, perfuming and preserving agents. The compositions according to the invention, for oral administration, also include capsules of absorbable material such as gelatin containing one or more of the active substances with or without the addition of diluents or excipients.

Solid compositions for vaginal administration include pessaries formulated in manner known per se and containing one or more of the active compounds.

Solid compositions for rectal administration include suppositories formulated in manner known per se and containing one or more of the active compounds.

Preparations according to the invention for parenteral administration include sterile aqueous or non-aqueous solutions, suspensions, or emulsions. Examples of non-aqueous solvents or suspending media are polyethylene glycol, dimethyl sulphoxide, vegetable oils such as olive oil and arachis oil, and injectable organic esters such as ethyl oleate. These compositions may also include adjuvants such as preserving, wetting, emulsifying and dispersing agents. They may be sterilised, for example, by filtration through a bacteria-retaining filter, by incorporation of sterilising agents in the compositions, or by irradiation. They may also be manufactured in the form of sterile solid compositions, which can be dissolved in sterile water or some other sterile injectable medium immediately before use.

The percentage of active ingredient in the compositions of the invention may be varied, it being necessary that it should constitute a proportion such that a suitable dosage for the therapeutic effect desired shall be obtained. Obviously several unit dosage forms may be administered at about the same time. In general, the preparations should normally contain at least 0.025% by weight of active substance when rquired for administration by injection, including administration by infusion; for oral administration the preparation will normally contain at least 0.1% by weight of active substance. The dose employed depends upon the desired therapeutic effect, the route of administration and the duration of the treatment.

The tetrazine derivatives of the general formula shown in Figure I are useful in the treatment of malignant neoplasms, for example carcinomas, melanomas, sarcomas, lymphomas and leukaemias, and in the treatment of organ grafts and skin grafts and in the treatment of immunological diseases at doses which are generally between 0.1 and 200, preferably between 1 and 20, mg/kg body weight per day preferably by intravenous administration, to ameliorate the condition of a patient. The treatment is useful to prevent or reduce the growth of malignant neoplasms.

The following Composition Examples illustrate pharmaceutical compositions according to the present invention.

## COMPOSITION EXAMPLE 1

A solution suitable for parenteral administration was prepared from the following ingredients:-

8-carboxy-3-(2-chloroethyl)-[3H]-imidazo[5,1-d]-1,2,3,5-tetrazin-4-one 1.0g
dimethyl sulphoxide 10ml
arachis oil 90ml

by dissolving the 8-carboxy-3-(2-chloroethyl)-[3 H]-imidazo[5,1-d]-1,2,3,5-tetrazin-4-one in the dimethyl sulphoxide and adding the arachis oil. The resulting solution was divided, under aseptic conditions, into ampoules at an amount of 10ml per ampoule. The ampoules were sealed, to give 10 ampoules each containing 100mg of 8-carboxy-3-(2-chloroethyl)-[3H]-imidazo[5,1-d]-1,2,3,5-tetrazin-4-one.

COMPOSITION EXAMPLE 2

A solution suitable for parenteral administration was prepared from the following ingredients:-
8-ethoxycarbonyl-3-methyl-[3H]-pyrazolo[5,1-d]-1,2,3,5-tetrazin-4-one 1.0g
dimethyl sulphoxide 10ml
arachis oil 90ml
by dissolving the 8-ethoxycarbonyl-3-methyl-[3 H]-pyrazolo[5,1-d]-1,2,3,5-tetrazin-4-one in the dimethyl sulphoxide and adding the arachis oil. The resulting solution was divided, under aseptic conditions, into ampoules at an amount of 10ml per ampoule. The ampoules were sealed, to give 10 ampoules each containing 100mg of 8-ethoxycarbonyl-3-methyl-[3H]-pyrazolo[5,1-d]-1,2,3,5-tetrazin-4-one .

Similar ampoules containing solutions suitable for parenteral administration may be prepared by proceeding in a similar manner but replacing the 8-ethoxycarbonyl-3-methyl-[3H]-pyrazolo[5,1-d]-1,2,3,5-tetrazin-4-one by another compound of the general formula shown in Figure I, for example compound E, G, K, N or Q.

COMPOSITION EXAMPLE 3

Capsules suitable for oral administration were prepared by placing 8-ethoxycarbonyl-3-methyl-[3H ]-pyrazolo[5,1-d]-1,2,3,5-tetrazin-4-one into gelatin shells of number 2 size at a rate of 10mg per capsule.

COMPOSITION EXAMPLE 4

Capsules suitable for oral administration were prepared by placing 8-carboxy-3-(2-chloroethyl)-[3H]-imidazo[5,1-d]-1,2,3,5-tetrazin-4-one into gelatin shells of number 2 size at a rate of 10mg per capsule.

Similar capsules may be prepared by using another compound of the general formula shown in Figure I, for example compound E, G, K, N or Q, or any other conveniently sized capsule shells.

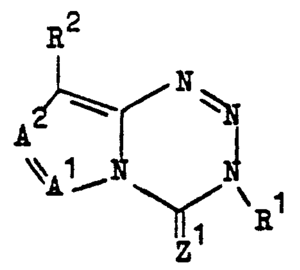

FIG. I

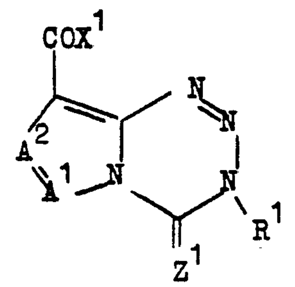

FIG. X

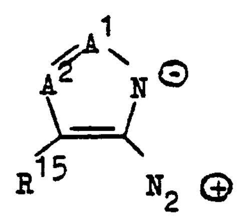

FIG. XI

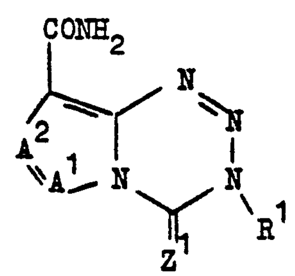

FIG.XIII

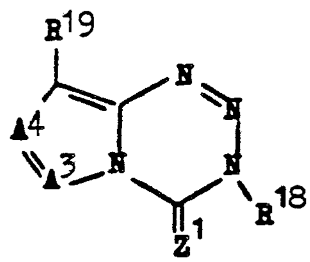

FIG. XXIII

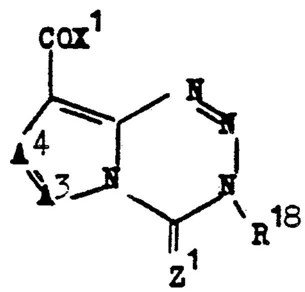

FIG. XXIV

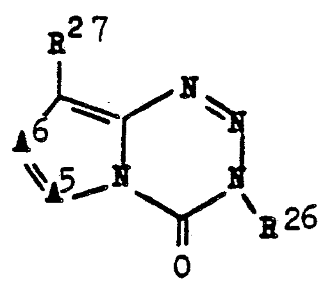

FIG. XXVI

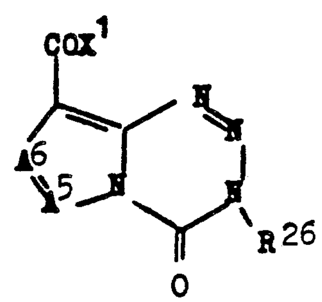

FIG. XXVII

## Claims

1. Use for the preparation of a pharmaceutical product of a tetrazine derivative of the general formula:

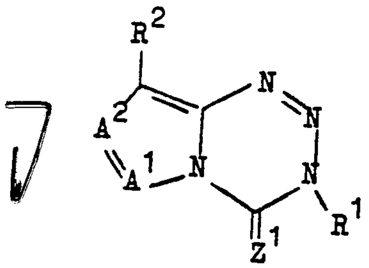

I

wherein $R^1$ represents a cycloalkyl group, or a straight-or branched-chain alkyl, alkenyl or alkynyl group containing up to 6 carbon atoms, each such alkyl, alkenyl or alkynyl group being unsubstituted or substituted by from one to three substituents selected from halogen atoms, straight-or branched-chain alkoxy, alkylthio, alkylsulphinyl and alkylsulphcnyl groups each containing up to 4 carbon atoms, and optionally substituted phenyl groups, $A^1$ represents a nitrogen atom or a group $-CR^3=$ wherein $R^3$ represents a hydrogen atom or a substituent $R^4$ wherein $R^4$ represents a halogen atom, or a straight-or branched-chain alkyl or alkenyl group, containing up to 6 carbon atoms, which may carry up to 3 substituents selected from halogen atoms, optionally substituted phenyl groups, and straight-or branched-chain alkoxy, alkylthio and alkylsulphonyl groups containing up to 3 carbon atoms, or $R^4$ represents a cycloalkyl, cyano, hydroxy, nitro or optionally substituted phenoxy group or a group of the formula $-COR^5$ - (wherein $R^5$ represents an alkyl or alkoxy group of up to 4 carbon atoms, or a hydroxy group, or an optionally substituted phenyl group) or an alkanoylamino group containing up to 6 carbon atoms, or $R^4$ represents a group of the formula $-S(O)_nR^6$, $-SO_2NR^7R^8$ or $-CZ^2NR^7R^8$ (wherein <u>n</u> represents 0, 1 or 2, $R^6$ represents a straight-or branched-chain alkyl or alkenyl group, containing up to 4 carbon atoms, which may carry an optionally substituted phenyl substituent, a cycloalkyl group or an optionally substituted phenyl group, $R^7$ and $R^8$, which may be the same or different, each represents a hydrogen atom or a straight-or branched-chain alkyl or alkenyl group, containing up to 4 carbon atoms, which may carry an optionally substituted phenyl substituent, or a cycloalkyl group or an optionally substituted phenyl group or the group $-NR^7R^8$ represents a heterocyclic group, and $Z^2$ represents an oxygen or sulphur atom), $A^2$ represents a nitrogen atom or, when $A^1$ represents a nitrogen atom, $A^2$ represents a nitrogen atom or a group $-CR^3=$ wherein $R^3$ is as hereinbefore defined, $Z^1$ represents an oxygen or sulphur atom, and $R^2$ represents a cyano or azidocarbonyl group or a group of the formulae II to IX:-

$-COZ^2R^9$ II
$-CONHOR^9$ III
$-CONHNR^9R^{10}$ IV
$-CONHNHCOOR^9$ V
$-CONHNHCONHR^9$ VI
$-COCR^{11}=N_2$ VII
$-CONHR^{12}$ VIII or
$-CON=S(O)R^{13}R^{14}$ IX

wherein $Z^2$ is as hereinbefore defined and $R^9$ and $R^{10}$ each represents a hydrogen atom or a straight-or branched-chain alkyl group containing from 1 to 4 carbon atoms, which may carry an optionally substituted phenyl substituent, or represents an optionally substituted phenyl group, $R^{11}$ represents a hydrogen atom or a straight-or branched-chain alkyl group containing from 1 to 4 carbon atoms, and $R^{12}$ represents a straight-or branched-chain alkyl group, containing from 1 to 4 carbon atoms, substituted by one or more halogen atoms or by a straight-or branched-chain alkoxycarbonyl group containing from 1 to 4 carbon atoms in the alkoxy moiety, or represents a straight-or branched-chain alkyl group containing 5 to 8 carbon atoms optionally substituted by one or more halogen atoms and $R^{13}$ and $R^{14}$ each represent a straight or branched chain alkyl group containing from 1 to 4 carbon atoms, and when $R^1$, $R^2$, or $R^3$ represents or contains an acidic group, salts thereof.

2. Use according to claim 1 for the preparation of a pharmaceutical product for the treatment of the human or animal body against carcinomas, melanomas, sarcomas, lymphomas and leukaemias, or for the treatment of organ or skin grafts or for the treatment of immunological diseases.

3. A tetrazine derivative as defined in claim 1 with the exclusion of compounds wherein $R^1$ represents a cycloalkyl group, or a straight-or branched-chain alkyl group containing up to 6 carbon atoms, optionally substituted by halogen or alkoxy containing up to 3 carbon atoms, or represents a methyl or ethyl group substituted by a phenyl group, $R^2$ represents a cyano group or an alkoxycarbonyl group containing up to 5 carbon atoms, $A^1$ represents a nitrogen atom or a group $-CR^3=$ wherein $R^3$ represents a hydrogen atom or a substituent $R^4$ wherein $R^4$ represents an alkyl, cycloalkyl or benzyl group, $A^2$ represents a nitrogen atom or a group $-CR^3=$ wherein $R^3$ represents a hydrogen atom or a substituent $R^4$ wherein $R^4$ represents an alkyl, cycloalkyl or benzyl group, and $Z^1$ represents an oxygen atom.

4. A tetrazine derivative according to claim 3 wherein $R^1$ represents the 2-chloroethyl group.

5. A tetrazine derivative according to claim 3 hereinbefore identified as any one of compounds A to G, K, N to W and AA.

6. A process for the preparation of a tetrazine derivative according to claim 3 which comprises the reaction of a compound of the general formula:

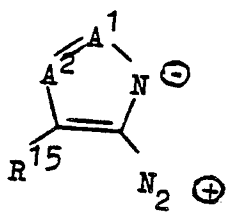

XI

wherein $A^1$ and $A^2$ are as defined in claim 3 and $R^{15}$ represents a group within the definition of $R^2$, with a compound of the general formula:-

$R^1NCZ^1$ XII

wherein $R^1$ and $Z^1$ are as defined in claim 3, optionally followed, when the tetrazine derivative obtained is a compound wherein $R^1$, $R^2$ or $R^3$ represents or contains an acidic group, by the step of converting the tetrazine derivative into a salt thereof.

7. A process according to claim 6 wherein $R^2$ and $R^{15}$ each represent a cyano group or an optionally substituted alkoxycarbonyl group.

8. A process for the preparation of a tetrazine derivative according to claim 3 wherein $R^2$ represents a carboxy or hydrocarbamoyl group and $R^1$, $A^1$, $A^2$ and $Z^1$ are as defined in claim 3, which comprises the debenzylation of a corresponding compound of general formula I, wherein $R^2$ represents a group of the formula $-COOR^{16}$ or $-CONHOR^{16}$ (wherein $R^{16}$ represents an optionally substituted benzyl group) to replace the optionally substituted benzyl group by a hydrogen atom, optionally followed, when the tetrazine derivative obtained is a compound wherein $R^1$, $R^2$ or $R^3$ represents or contains an acidic group, by the step of converting the tetrazine derivative into a salt thereof.

9. A process for the preparation of a tetrazine derivative according to claim 3 wherein $R^2$ represents a carboxy group which comprises the conversion to a carboxy group of the group $CONH_2$ of a compound of the general formula:

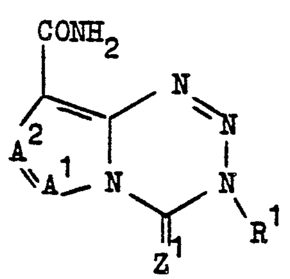

XIII

wherein $A^1$, $A^2$, $R^1$ and $Z^1$ are as defined in claim 3, optionally followed, when the tetrazine derivative obtained is a compound wherein $R^1$, $R^2$ or $R^3$ represents or contains an acidic group, by the step of converting the tetrazine derivative into a salt thereof.

10. A process for the preparation of a tetrazine derivative according to claim 3 wherein $R^2$ is other than a cyano group which comprises the reaction of a compound of the general formula:

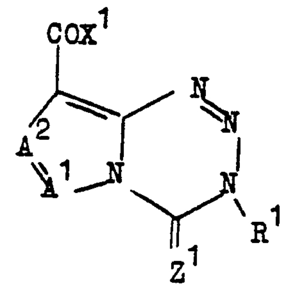

X

wherein $A^1$, $A^2$, $R^1$, $X^1$ and $Z^1$ are as defined in claim 3 with a compound of the formulae XIV to XXII:-

$MN_3$ XIV

$R^{17}Z^2R^9$ XV

$NH_2OR^9$ XVI

$NH_2NR^9R^{10}$ XVII

$NH_2NHCOOR^9$ XVIII

$NH_2NHCONHR^9$ XIX

$CHR^{11} = N_2$ XX

$NH_2R^{12}$ XXI

$HN = S(O)R^{13}R^{14}$ XXII

wherein $R^9$, $R^{10}$, $R^{11}$, $R^{12}$, $R^{13}$, $R^{14}$ and $Z^2$ are as defined in claim 3, M represents an alkali metal atom and $R^{17}$ represents a hydrogen atom or an alkali metal atom, optionally followed, when the tetrazine derivative obtained is a compound wherein $R^1$, $R^2$ or $R^3$ represents or contains an acidic group, by the step of converting the tetrazine derivative into a salt thereof.

11. A pharmaceutical composition which comprises, as active ingredient, a tetrazine derivative according to claim 3 or a pharmaceutically acceptable salt thereof in association with a pharmaceutical carrier or coating.

12. An acid halide of the general formula:

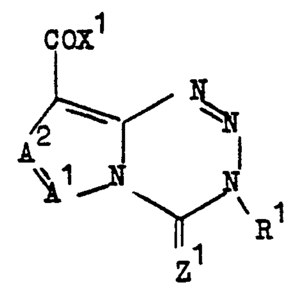

X

wherein $A^1$, $A^2$, $R^1$ and $Z^1$ are as defined in claim 1 and $X^1$ represents a halogen atom.

13. A process for the preparation of a compound according to claim 12 which comprises the conversion to a group $-COX^1$ of the carboxy group of a compound of the general formula depicted in claim 1 wherein $R^2$ represents a carboxy group and $A^1$, $A^2$, $R^1$ and $Z^1$ are as defined in claim 1.

14. A process for the preparation of a tetrazine derivative of the general formula:

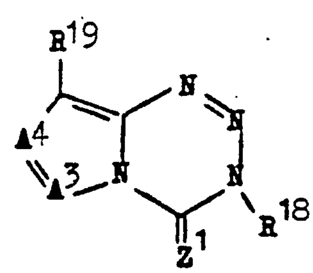

XXIII

wherein $R^{18}$ represents a cycloalkyl group, or a straight-or branched-chain alkyl, alkenyl or alkynyl group containing up to 6 carbon atoms, each such alkyl, alkenyl or alkynyl group being unsubstituted or

substituted by from one to three substituents selected from halogen atoms, straight-or branched-chain alkoxy, alkylthio, alkylsulphinyl and alkylsulphonyl groups each containing up to 4 carbon atoms, and optionally substituted phenyl groups, $A^3$ represents a nitrogen atom or a group $-CR^{20}=$ wherein $R^{20}$ represents a hydrogen atom or a substituent $R^{21}$ wherein $R^{21}$ represents a halogen atom, or a straight-or branched-chain alkyl or alkenyl group, containing up to 6 carbon atoms, which may carry up to 3 substituents selected from halogen atoms, optionally substituted phenyl groups, and straight-or branched-chain alkoxy, alkylthio and alkylsulphonyl groups containing up to 3 carbon atoms, or $R^{21}$ represents a cycloalkyl, cyano, nitro or optionally substituted phenoxy groups or a group of the formula $-COR^{22}$ (wherein $R^{22}$ represents an alkyl or alkoxy group of up to 4 carbon atoms or an optionally substituted phenyl group), or $R^{21}$ represents a group of the formula:-

$-S(O)_nR^{23}$, $-SO_2NR^{24}R^{25}$ or $-CONR^{24}R^{25}$

(wherein n represents 0 or 2, $R^{23}$ represents a straight-or branched-chain alkyl or alkenyl group, containing up to 4 carbon atoms, which may carry an optionally substituted phenyl substituent, a cycloalkyl group or an optionally substituted phenyl group, $R^{24}$ and $R^{25}$, which may be the same or different, each represent a straight-or branched-chain alkyl or alkenyl group, containing up to 4 carbon atoms, which may carry an optionally substituted phenyl substituent, or a cycloalkyl group or an optionally substituted phenyl group or the group $-NR^{24}R^{25}$ represents a heterocyclic group, $A^4$ represents a nitrogen atom or, when $A^3$ represents a nitrogen atom, $A^4$ represents a nitrogen atom or a group $-CR^3=$ wherein $R^3$ is as hereinbefore defined, $Z^1$ represents an oxygen or sulphur atom, and $R^2$ represents a group of the formula:-

$-CONR^{24}R^{25}$

(wherein $R^{24}$ and $R^{25}$, which may be the same or different, each represents a straight-or branched-chain alkyl or alkenyl group, containing up to 4 carbon atoms, which may carry an optionally substituted phenyl substituent, or a cycloalkyl group or an optionally substituted phenyl group or the group $-NR^{24}R^{25}$ represents a heterocyclic group) which comprises the reaction of a compound of the general formula:

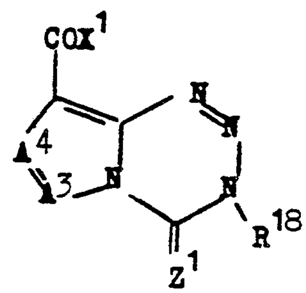

XXIV

wherein $A^3$, $A^4$, $R^{18}$, $X^1$ and $Z^1$ are as hereinbefore defined with a compound of the general formula:

$HNR^{24}R^{25}$ XXV

wherein $R^{24}$ and $R^{25}$ are as hereinbefore defined.

15. A process for the preparation of a tetrazine derivative of the general formula:

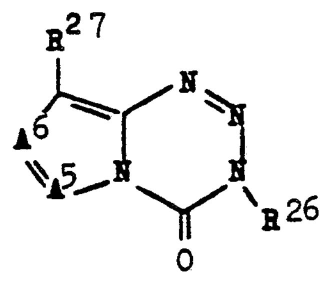

XXVI

wherein $R^{26}$ represents a cycloalkyl group, or a straight-or branched-chain alkyl group containing up to 6 carbon atoms, optionally substituted by halogen or alkoxy containing up to 3 carbon atoms, or represents a methyl or ethyl group substituted by a phenyl group, $R^{27}$ represents an alkoxycarbonyl group containing up to 5 carbon atoms, $A^5$ represents a nitrogen atom or a group $-CR^{28}=$ wherein $R^{28}$ represents a hydrogen atom or a substituent $R^{29}$ wherein $R^{29}$ represents an alkyl, cycloalkyl or benzyl group, $A^6$ represents a nitrogen atom or a group $-CR^{28}=$ wherein $R^{28}$ represents a hydrogen atom or a substituent $R^{29}$ wherein $R^{29}$ represents an alkyl, cycloalkyl or benzyl group, which comprises the reaction of a compound of the general

formula:

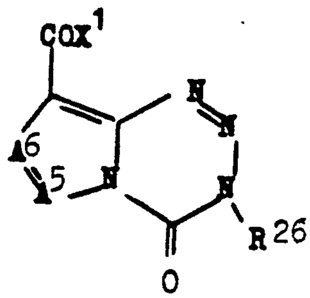

XXVII

wherein $A^5$, $A^6$ and $R^{26}$ are as hereinbefore defined and $X^1$ is as defined in claim 16, with a compound of the general formula:

$R^{30}OH$ XXVIII

wherein $R^{30}$ represents an alkyl group containing up to 4 carbon atoms.